# EUROPEAN PATENT SPECIFICATION

(11) **EP 2 055 308 B1**
(45) Date of publication and mention of the grant of the patent: **10.05.2017**
(21) Application number: 07830867.3
(22) Date of filing: 30.10.2007
(51) Int. Cl.: A61K 38/00, A61K 31/711, A61K 35/12, A61K 35/76, A61K 48/00, A61P 1/04, A61P 9/10, A61P 11/00, A61P 17/00, A61P 17/02, A61P 17/14

(54) **PHARMACEUTICAL FOR PROMOTING FUNCTIONAL REGENERATION OF DAMAGED TISSUE**
ARZNEIMITTEL ZUR FÖRDERUNG DER FUNKTIONALEN NEUBILDUNG VON BESCHÄDIGTEM GEWEBE
PRODUIT PHARMACEUTIQUE SERVANT À FAVORISER LA RÉGÉNÉRATION FONCTIONNELLE D'UN TISSU ENDOMMAGÉ

(30) Priority: 30.10.2006 JP 2006293582
(43) Date of publication of application: 06.05.2009
(73) Proprietor: Genomix Co., Ltd., Osaka 567-0085 (JP); Osaka University, Suita-shi Osaka 565-0871 (JP)
(72) Inventor: TAMAI, Katsuto, Suita-shi Osaka 5650871 (JP); YAMAZAKI, Takehiko, Minoo-shi Osaka 5620031 (JP); KANEDA, Yasufumi, Suita-shi Osaka 5650871 (JP)
(74) Representative: Zacco GmbH
(86) International application number: PCT/JP2007/071133
(87) International publication number: WO 2008/053892

(56) References cited:
- WO-A1-2007/015546
- WO-A2-2004/004763
- WO-A2-2004/061456
- WO-A2-2005/025604
- JP-A- 2003 505 506
- JP-A- 2005 508 913
- JP-A- 2005 537 253
- JP-A- 2006 124 389
- JP-A- 2006 517 537
- US-A1- 2004 053 841
- US-A1- 2006 035 851
- PALUMBO ROBERTA ET AL: "Extracellular HMGB1, a signal of tissue damage, induces mesoangioblast migration and proliferation." THE JOURNAL OF CELL BIOLOGY 2 FEB 2004 LNKD- PUBMED:14744997, vol. 164, no. 3, 2 February 2004 (2004-02-02), pages 441-449, XP002600991 ISSN: 0021-9525
- PALUMBO ROBERTA ET AL: "High mobility group box 1 protein, a cue for stem cell recruitment." BIOCHEMICAL PHARMACOLOGY 15 SEP 2004 LNKD- PUBMED:15313414, vol. 68, no. 6, 15 September 2004 (2004-09-15), pages 1165-1170, XP002600992 ISSN: 0006-2952
- TAGLIAFICO ENRICO ET AL: "TGFbeta/BMP activate the smooth muscle/bone differentiation programs in mesoangioblasts.", JOURNAL OF CELL SCIENCE 1 SEP 2004, vol. 117, no. Pt 19, 1 September 2004 (2004-09-01), pages 4377-4388, ISSN: 0021-9533

### SEQUENCE LISTING

<110> GENOMIX CO., LTD.
<120> Pharmaceutical for promoting functional regeneration of damaged tissue
<130> G 10013 EP
<140> EP 07830867.3
   <141> 2007-10-30
<150> JP 2006-293582
   <151> 2006-10-30
<160> 18
<170> PatentIn version 3.4
<210> 1
   <211> 215
   <212> PRT
   <213> Homo sapiens
<400> 1
   Met Gly Lys Gly Asp Pro Lys Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ala Ser Val Asn Phe Ser Glu Phe Ser Lys Lys Cys Ser Glu Arg
           35                  40                  45
   Trp Lys Thr Met Ser Ala Lys Glu Lys Gly Lys Phe Glu Asp Met Ala
       50                  55                  60
   Lys Ala Asp Lys Ala Arg Tyr Glu Arg Glu Met Lys Thr Tyr Ile Pro
   65                  70                  75                  80
   Pro Lys Gly Glu Thr Lys Lys Lys Phe Lys Asp Pro Asn Ala Pro Lys
                   85                  90                  95
   Arg Pro Pro Ser Ala Phe Phe Leu Phe Cys Ser Glu Tyr Arg Pro Lys
               100                 105                 110
   Ile Lys Gly Glu His Pro Gly Leu Ser Ile Gly Asp Val Ala Lys Lys
           115                 120                 125
   Leu Gly Glu Met Trp Asn Asn Thr Ala Ala Asp Asp Lys Gln Pro Tyr
       130                 135                 140
   Glu Lys Lys Ala Ala Lys Leu Lys Glu Lys Tyr Glu Lys Asp Ile Ala
   145                 150                 155                 160
   Ala Tyr Arg Ala Lys Gly Lys Pro Asp Ala Ala Lys Lys Gly Val Val
                   165                 170                 175
   Lys Ala Glu Lys Ser Lys Lys Lys Lys Glu Glu Glu Glu Asp Glu Glu
               180                 185                 190
   Asp Glu Glu Asp Glu Glu Glu Glu Glu Asp Glu Glu Asp Glu Asp Glu
           195                 200                 205
   Glu Glu Asp Asp Asp Asp Glu
       210                 215
<210> 2
   <211> 648
   <212> DNA
   <213> Homo sapiens
<400> 2
   atgggcaaag gagatcctaa gaagccgaga ggcaaaatgt catcatatgc attttttgtg      60
   caaacttgtc gggaggagca taagaagaag cacccagatg cttcagtcaa cttctcagag     120
   ttttctaaga agtgctcaga gaggtggaag accatgtctg ctaaagagaa aggaaaattt     180
   gaagatatgg caaaagcgga caaggcccgt tatgaaagag aaatgaaaac ctatatccct     240
   cccaaagggg agacaaaaaa gaagttcaag gatcccaatg cacccaagag gcctccttcg     300
   gccttcttcc tcttctgctc tgagtatcgc ccaaaaatca aaggagaaca tcctggcctg     360
   tccattggtg atgttgcgaa gaaactggga gagatgtgga ataacactgc tgcagatgac     420
   aagcagcctt atgaaaagaa ggctgcgaag ctgaaggaaa aatacgaaaa ggatattgct     480
   gcatatcgag ctaaaggaaa gcctgatgca gcaaaaaagg gagttgtcaa ggctgaaaaa     540
   agcaagaaaa agaaggaaga ggaggaagat gaggaagatg aagaggatga ggaggaggag     600
   gaagatgaag aagatgaaga tgaagaagaa gatgatgatg atgaataa                   648
<210> 3
   <211> 215
   <212> PRT
   <213> Mus musculus
<400> 3
   Met Gly Lys Gly Asp Pro Lys Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ala Ser Val Asn Phe Ser Glu Phe Ser Lys Lys Cys Ser Glu Arg
           35                  40                  45
   Trp Lys Thr Met Ser Ala Lys Glu Lys Gly Lys Phe Glu Asp Met Ala
       50                  55                  60
   Lys Ala Asp Lys Ala Arg Tyr Glu Arg Glu Met Lys Thr Tyr Ile Pro
   65                  70                  75                  80
   Pro Lys Gly Glu Thr Lys Lys Lys Phe Lys Asp Pro Asn Ala Pro Lys
                   85                  90                  95
   Arg Pro Pro Ser Ala Phe Phe Leu Phe Cys Ser Glu Tyr Arg Pro Lys
               100                 105                 110
   Ile Lys Gly Glu His Pro Gly Leu Ser Ile Gly Asp Val Ala Lys Lys
           115                 120                 125
   Leu Gly Glu Met Trp Asn Asn Thr Ala Ala Asp Asp Lys Gln Pro Tyr
       130                 135                 140
   Glu Lys Lys Ala Ala Lys Leu Lys Glu Lys Tyr Glu Lys Asp Ile Ala
   145                 150                 155                 160
   Ala Tyr Arg Ala Lys Gly Lys Pro Asp Ala Ala Lys Lys Gly Val Val
                   165                 170                 175
   Lys Ala Glu Lys Ser Lys Lys Lys Lys Glu Glu Glu Asp Asp Glu Glu
               180                 185                 190
   Asp Glu Glu Asp Glu Glu Glu Glu Glu Glu Glu Glu Asp Glu Asp Glu
           195                 200                 205
   Glu Glu Asp Asp Asp Asp Glu
       210                 215
<210> 4
   <211> 648
   <212> DNA
   <213> Mus musculus
<400> 4
   atgggcaaag gagatcctaa aaagccgaga ggcaaaatgt cctcatatgc attctttgtg     60
   caaacttgcc gggaggagca caagaagaag cacccggatg cttctgtcaa cttctcagag    120
   ttctccaaga agtgctcaga gaggtggaag accatgtctg ctaaagaaaa ggggaaattt    180
   gaagatatgg caaaggctga caaggctcgt tatgaaagag aaatgaaaac ctacatcccc    240
   cccaaagggg agaccaaaaa gaagttcaag gaccccaatg cacccaagag gcctccttcg    300
   gccttcttct tgttctgttc tgagtaccgc cccaaaatca aaggcgagca tcctggctta    360
   tccattggtg atgttgcaaa gaaactagga gagatgtgga acaacactgc agcagatgac    420
   aagcagccct atgagaagaa agctgccaag ctgaaggaga agtatgagaa ggatattgct    480
   gcctacagag ctaaaggaaa acctgatgca gcgaaaaagg gggtggtcaa ggctgaaaag    540
   agcaagaaaa agaaggaaga ggaagatgat gaggaggatg aagaggatga ggaagaggag    600
   gaagaagagg aagacgaaga tgaagaagaa gatgatgatg atgaataa                 648
<210> 5
   <211> 215
   <212> PRT
   <213> Rattus norvegicus
<400> 5
   Met Gly Lys Gly Asp Pro Lys Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ala Ser Val Asn Phe Ser Glu Phe Ser Lys Lys Cys Ser Glu Arg
           35                  40                  45
   Trp Lys Thr Met Ser Ala Lys Glu Lys Gly Lys Phe Glu Asp Met Ala
       50                  55                  60
   Lys Ala Asp Lys Ala Arg Tyr Glu Arg Glu Met Lys Thr Tyr Ile Pro
   65                  70                  75                  80
   Pro Lys Gly Glu Thr Lys Lys Lys Phe Lys Asp Pro Asn Ala Pro Lys
                   85                  90                  95
   Arg Pro Pro Ser Ala Phe Phe Leu Phe Cys Ser Glu Tyr Arg Pro Lys
               100                 105                 110
   Ile Lys Gly Glu His Pro Gly Leu Ser Ile Gly Asp Val Ala Lys Lys
           115                 120                 125
   Leu Gly Glu Met Trp Asn Asn Thr Ala Ala Asp Asp Lys Gln Pro Tyr
       130                 135                 140
   Glu Lys Lys Ala Ala Lys Leu Lys Glu Lys Tyr Glu Lys Asp Ile Ala
   145                 150                 155                 160
   Ala Tyr Arg Ala Lys Gly Lys Pro Asp Ala Ala Lys Lys Gly Val Val
                   165                 170                 175
   Lys Ala Glu Lys Ser Lys Lys Lys Lys Glu Glu Glu Asp Asp Glu Glu
               180                 185                 190
   Asp Glu Glu Asp Glu Glu Glu Glu Glu Glu Glu Glu Asp Glu Asp Glu
           195                 200                 205
   Glu Glu Asp Asp Asp Asp Glu
       210                 215
<210> 6
   <211> 648
   <212> DNA
   <213> Rattus norvegicus
<400> 6
   atgggcaaag gagatcctaa gaagccgaga ggcaaaatgt cctcatatgc attctttgtg      60
   caaacctgcc gggaggagca caagaagaag cacccggatg cttctgtcaa cttctcagag     120
   ttctccaaga agtgctcaga gaggtggaag accatgtctg ctaaagaaaa ggggaaattt     180
   gaagatatgg caaaggctga caaggctcgt tatgaaagag aaatgaaaac ctacatcccc     240
   cccaaagggg agaccaaaaa gaagttcaag gaccccaatg cccccaagag gcctccttcg     300
   gccttcttct tgttctgttc tgagtaccgc ccaaaaatca aaggcgagca tcctggctta     360
   tccattggtg atgttgcgaa gaaactagga gagatgtgga acaacactgc tgcggatgac     420
   aagcagccct atgaaaagaa ggccgccaag ctgaaggaga agtatgagaa ggatattgct     480
   gcctacagag ctaaaggaaa acctgatgca gcgaaaaagg gggtggtcaa ggctgagaag     540
   agcaagaaaa agaaggaaga ggaagacgac gaggaggatg aagaggatga ggaagaggag     600
   gaagaggagg aagacgaaga tgaagaagaa gatgatgatg atgaataa                  648
<210> 7
   <211> 209
   <212> PRT
   <213> Homo sapiens
<400> 7
   Met Gly Lys Gly Asp Pro Asn Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ser Ser Val Asn Phe Ala Glu Phe Ser Lys Lys Cys Ser Glu Arg
           35                  40                  45
   Trp Lys Thr Met Ser Ala Lys Glu Lys Ser Lys Phe Glu Asp Met Ala
   50 55 60
   Lys Ser Asp Lys Ala Arg Tyr Asp Arg Glu Met Lys Asn Tyr Val Pro
   65 70 75 80
   Pro Lys Gly Asp Lys Lys Gly Lys Lys Lys Asp Pro Asn Ala Pro Lys
   85 90 95
   Arg Pro Pro Ser Ala Phe Phe Leu Phe Cys Ser Glu His Arg Pro Lys
   100 105 110
   Ile Lys Ser Glu His Pro Gly Leu Ser Ile Gly Asp Thr Ala Lys Lys
   115 120 125
   Leu Gly Glu Met Trp Ser Glu Gln Ser Ala Lys Asp Lys Gln Pro Tyr
   130 135 140
   Glu Gln Lys Ala Ala Lys Leu Lys Glu Lys Tyr Glu Lys Asp Ile Ala
   145 150 155 160
   Ala Tyr Arg Ala Lys Gly Lys Ser Glu Ala Gly Lys Lys Gly Pro Gly
   165 170 175
   Arg Pro Thr Gly Ser Lys Lys Lys Asn Glu Pro Glu Asp Glu Glu Glu
   180 185 190
   Glu Glu Glu Glu Glu Asp Glu Asp Glu Glu Glu Glu Asp Glu Asp Glu
   195 200 205
   Glu
<210> 8
   <211> 630
   <212> DNA
   <213> Homo sapiens
<400> 8
   atgggtaaag gagaccccaa caagccgcgg ggcaaaatgt cctcgtacgc cttcttcgtg 60
   cagacctgcc gggaagagca caagaagaaa cacccggact cttccgtcaa tttcgcggaa 120
   ttctccaaga agtgttcgga gagatggaag accatgtctg caaaggagaa gtcgaagttt 180
   gaagatatgg caaaaagtga caaagctcgc tatgacaggg agatgaaaaa ttacgttcct 240
   cccaaaggtg ataagaaggg gaagaaaaag gaccccaatg ctcctaaaag gccaccatct 300
   gccttcttcc tgttttgctc tgaacatcgc ccaaagatca aaagtgaaca ccctggccta 360
   tccattgggg atactgcaaa gaaattgggt gaaatgtggt ctgagcagtc agccaaagat 420
   aaacaaccat atgaacagaa agcagctaag ctaaaggaga aatatgaaaa ggatattgct 480
   gcatatcgtg ccaagggcaa aagtgaagca ggaaagaagg gccctggcag gccaacaggc 540
   tcaaagaaga agaacgaacc agaagatgag gaggaggagg aggaagaaga agatgaagat 600
   gaggaggaag aggatgaaga tgaagaataa 630
<210> 9
   <211> 210
   <212> PRT
   <213> Mus musculus
<400> 9
   Met Gly Lys Gly Asp Pro Asn Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ser Ser Val Asn Phe Ala Glu Phe Ser Lys Lys Cys Ser Glu Arg
           35                  40                  45
   Trp Lys Thr Met Ser Ala Lys Glu Lys Ser Lys Phe Glu Asp Leu Ala
       50                  55                  60
   Lys Ser Asp Lys Ala Arg Tyr Asp Arg Glu Met Lys Asn Tyr Val Pro
   65                  70                  75                  80
   Pro Lys Gly Asp Lys Lys Gly Lys Lys Lys Asp Pro Asn Ala Pro Lys
                   85                  90                  95
   Arg Pro Pro Ser Ala Phe Phe Leu Phe Cys Ser Glu Asn Arg Pro Lys
               100                 105                 110
   Ile Lys Ile Glu His Pro Gly Leu Ser Ile Gly Asp Thr Ala Lys Lys
           115                 120                 125
   Leu Gly Glu Met Trp Ser Glu Gln Ser Ala Lys Asp Lys Gln Pro Tyr
       130                 135                 140
   Glu Gln Lys Ala Ala Lys Leu Lys Glu Lys Tyr Glu Lys Asp Ile Ala
   145                 150                 155                 160
   Ala Tyr Arg Ala Lys Gly Lys Ser Glu Ala Gly Lys Lys Gly Pro Gly
                   165                 170                 175
   Arg Pro Thr Gly Ser Lys Lys Lys Asn Glu Pro Glu Asp Glu Glu Glu
               180                 185                 190
   Glu Glu Glu Glu Glu Glu Glu Glu Asp Asp Glu Glu Glu Glu Glu Asp
           195                 200                 205
   Glu Glu
       210
<210> 10
   <211> 633
   <212> DNA
   <213> Mus musculus
<400> 10
   atgggcaagg gtgaccccaa caagccgcgg ggcaaaatgt cctcgtacgc cttcttcgtg     60
   cagacctgcc gcgaggagca caagaagaag catcccgact cgtcggtgaa cttcgccgag    120
   ttctccaaga aatgctccga gagatggaag accatgtctg caaaggaaaa gtccaagttt    180
   gaagatttgg ccaagagcga caaagctcgt tatgacaggg agatgaagaa ctatgttcct    240
   cccaaagggg ataagaaagg aaagaaaaaa gaccccaatg ctccgaagag accaccgtct    300
   gccttcttcc tgttttgctc tgaaaatcgc ccaaagatca aaattgaaca cccaggcctg    360
   tctattggag atactgcgaa gaaactgggt gagatgtggt ctgagcaatc tgccaaagat    420
   aaacaaccgt atgagcagaa agcagctaaa ctaaaggaga agtatgaaaa ggatattgct    480
   gcataccgtg ccaagggcaa aagtgaagca ggaaagaagg gtcctggtag gccaacaggc    540
   tcaaagaaga agaacgaacc agaagatgag gaggaggaag aagaggagga agaggaggaa    600
   gatgacgagg aagaagagga ggatgaagaa taa                                 633
<210> 11
   <211> 210
   <212> PRT
   <213> Rattus norvegicus
<400> 11
   Met Gly Lys Gly Asp Pro Asn Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ser Ser Val Asn Phe Ala Glu Phe Ser Lys Lys Cys Ser Glu Arg
           35                  40                  45
   Trp Lys Thr Met Ser Ala Lys Glu Lys Ser Lys Phe Glu Asp Leu Ala
       50                  55                  60
   Lys Ser Asp Lys Ala Arg Tyr Asp Arg Glu Met Lys Asn Tyr Val Pro
   65                  70                  75                  80
   Pro Lys Gly Asp Lys Lys Gly Lys Lys Lys Asp Pro Asn Ala Pro Lys
                   85                  90                  95
   Arg Pro Pro Ser Ala Phe Phe Leu Phe Cys Ser Glu His Arg Pro Lys
               100                 105                 110
   Ile Lys Ser Glu His Pro Gly Leu Ser Ile Gly Asp Thr Ala Lys Lys
           115                 120                 125
   Leu Gly Glu Met Trp Ser Glu Gln Ser Ala Lys Asp Lys Gln Pro Tyr
       130                 135                 140
   Glu Gln Lys Ala Ala Lys Leu Lys Glu Lys Tyr Glu Lys Asp Ile Ala
   145                 150                 155                 160
   Ala Tyr Arg Ala Lys Gly Lys Ser Glu Val Gly Lys Lys Gly Pro Gly
                   165                 170                 175
   Arg Pro Thr Gly Ser Lys Lys Lys Asn Glu Pro Glu Asp Glu Glu Glu
               180                 185                 190
   Glu Glu Glu Glu Glu Asp Asp Glu Asp Glu Glu Glu Glu Asp Glu Asp
           195                 200                 205
   Glu Glu
       210
<210> 12
   <211> 633
   <212> DNA
   <213> Rattus norvegicus
<400> 12
   atgggcaagg gggaccccaa caagccgcgg ggcaagatgt cctcgtacgc cttcttcgtg      60
   cagacctgcc gggaggagca caagaagaag catcccgact cgtcggtcaa cttcgccgag     120
   ttctcgaaga aatgttcgga gagatggaag accatgtctg ccaaggaaaa gtcgaagttt     180
   gaggatttgg ccaagagcga caaagctcgt tatgacaggg agatgaagaa ctatgttcct     240
   cccaaaggtg ataagaaagg aaagaaaaaa gatccaaatg ctcccaagag accaccgtct     300
   gccttcttcc tgttttgctc tgaacatcgc ccaaagatca aaagtgaaca ccccggcctg     360
   tctattggag atactgcaaa gaaactgggg gagatgtggt ctgagcaatc tgccaaagat     420
   aaacaaccgt atgagcagaa agcagctaaa ctaaaggaga agtatgaaaa ggatattgct     480
   gcataccgtg ccaagggcaa aagtgaagta ggaaagaagg gtcctggtag gccaacaggc     540
   tcaaagaaga agaatgaacc agaagatgag gaagaggagg aggaggaaga agatgatgaa     600
   gatgaagagg aggaagatga ggatgaagaa taa                                   633
<210> 13
   <211> 200
   <212> PRT
   <213> Homo sapiens
<400> 13
   Met Ala Lys Gly Asp Pro Lys Lys Pro Lys Gly Lys Met Ser Ala Tyr
   1               5                   10                  15
   Ala Phe Phe Val Gln Thr Cys Arg Glu Glu His Lys Lys Lys Asn Pro
               20                  25                  30
   Glu Val Pro Val Asn Phe Ala Glu Phe Ser Lys Lys Cys Ser Glu Arg
           35                  40                  45
   Trp Lys Thr Met Ser Gly Lys Glu Lys Ser Lys Phe Asp Glu Met Ala
       50                  55                  60
   Lys Ala Asp Lys Val Arg Tyr Asp Arg Glu Met Lys Asp Tyr Gly Pro
   65                  70                  75                  80
   Ala Lys Gly Gly Lys Lys Lys Lys Asp Pro Asn Ala Pro Lys Arg Pro
                   85                  90                  95
   Pro Ser Gly Phe Phe Leu Phe Cys Ser Glu Phe Arg Pro Lys Ile Lys
               100                 105                 110
   Ser Thr Asn Pro Gly Ile Ser Ile Gly Asp Val Ala Lys Lys Leu Gly
           115                 120                 125
   Glu Met Trp Asn Asn Leu Asn Asp Ser Glu Lys Gln Pro Tyr Ile Thr
       130                 135                 140
   Lys Ala Ala Lys Leu Lys Glu Lys Tyr Glu Lys Asp Val Ala Asp Tyr
   145                 150                 155                 160
   Lys Ser Lys Gly Lys Phe Asp Gly Ala Lys Gly Pro Ala Lys Val Ala
                   165                 170                 175
   Arg Lys Lys Val Glu Glu Glu Asp Glu Glu Glu Glu Glu Glu Glu Glu
               180                 185                 190
   Glu Glu Glu Glu Glu Glu Asp Glu
           195                 200
<210> 14
   <211> 603
   <212> DNA
   <213> Homo sapiens
<400> 14
   atggctaaag gtgaccccaa gaaaccaaag ggcaagatgt ccgcttatgc cttctttgtg     60
   cagacatgca gagaagaaca taagaagaaa aacccagagg tccctgtcaa ttttgcggaa    120
   ttttccaaga agtgctctga gaggtggaag acgatgtccg ggaaagagaa atctaaattt    180
   gatgaaatgg caaaggcaga taaagtgcgc tatgatcggg aaatgaagga ttatggacca    240
   gctaagggag gcaagaagaa gaaggatcct aatgctccca aaaggccacc gtctggattc    300
   ttcctgttct gttcagaatt ccgccccaag atcaaatcca caaaccccgg catctctatt    360
   ggagacgtgg caaaaaagct gggtgagatg tggaataatt taaatgacag tgaaaagcag    420
   ccttacatca ctaaggcggc aaagctgaag gagaagtatg agaaggatgt tgctgactat    480
   aagtcgaaag gaaagtttga tggtgcaaag ggtcctgcta aagttgcccg gaaaaaggtg    540
   gaagaggaag atgaagaaga ggaggaggaa gaagaggagg aggaggagga ggaggatgaa    600
   taa                                                                  603
<210> 15
   <211> 200
   <212> PRT
   <213> Mus musculus
<400> 15
   Met Ala Lys Gly Asp Pro Lys Lys Pro Lys Gly Lys Met Ser Ala Tyr
   1               5                   10                  15
   Ala Phe Phe Val Gln Thr Cys Arg Glu Glu His Lys Lys Lys Asn Pro
               20                  25                  30
   Glu Val Pro Val Asn Phe Ala Glu Phe Ser Lys Lys Cys Ser Glu Arg
           35                  40                  45
   Trp Lys Thr Met Ser Ser Lys Glu Lys Ser Lys Phe Asp Glu Met Ala
       50                  55                  60
   Lys Ala Asp Lys Val Arg Tyr Asp Arg Glu Met Lys Asp Tyr Gly Pro
   65                  70                  75                  80
   Ala Lys Gly Gly Lys Lys Lys Lys Asp Pro Asn Ala Pro Lys Arg Pro
                   85                  90                  95
   Pro Ser Gly Phe Phe Leu Phe Cys Ser Glu Phe Arg Pro Lys Ile Lys
               100                 105                 110
   Ser Thr Asn Pro Gly Ile Ser Ile Gly Asp Val Ala Lys Lys Leu Gly
           115                 120                 125
   Glu Met Trp Asn Asn Leu Ser Asp Asn Glu Lys Gln Pro Tyr Val Thr
       130                 135                 140
   Lys Ala Ala Lys Leu Lys Glu Lys Tyr Glu Lys Asp Val Ala Asp Tyr
   145                 150                 155                 160
   Lys Ser Lys Gly Lys Phe Asp Gly Ala Lys Gly Pro Ala Lys Val Ala
                   165                 170                 175
   Arg Lys Lys Val Glu Glu Glu Glu Glu Glu Glu Glu Glu Glu Glu Glu
               180                 185                 190
   Glu Glu Glu Glu Glu Glu Asp Glu
           195                 200
<210> 16
   <211> 603
   <212> DNA
   <213> Mus musculus
<400> 16
   atggctaaag gtgaccccaa gaaaccaaag ggcaagatgt ctgcttatgc cttctttgtg      60
   cagacatgca gggaagaaca taagaagaaa aacccagagg ttcccgtcaa ttttgctgag     120
   ttctccaaga agtgctcgga gaggtggaag accatgtcta gcaaagagaa atcaaagttt     180
   gatgaaatgg caaaggcaga taaagtccga tatgatcggg agatgaaaga ttatggacca     240
   gctaaaggag gcaagaagaa gaaggaccca aatgccccca aaagacctcc gtctggattt     300
   ttcttattct gctctgaatt ccgccccaag atcaaatcca caaaccctgg catctccatt     360
   ggagatgtgg caaaaaagct gggtgagatg tggaataact taagtgacaa tgaaaagcag     420
   ccttatgtca ccaaggcagc aaagctgaag gagaagtatg agaaggatgt tgctgactat     480
   aagtctaaag ggaagtttga tggtgccaag ggtcctgcta aagttgcccg gaaaaaggtg     540
   gaagaagagg aagaggagga ggaagaggaa gaagaggagg aggaagagga ggaagatgaa     600
   taa                                                                   603
<210> 17
   <211> 63
   <212> DNA
   <213> Artificial
<220>
   <223> An artificially synthesized nucleotide sequence
<400> 17
   atgcagacag acacactcct gctatgggta ctgctgctgt gggttccagg ttccactggt      60
   gac                                                                    63
<210> 18
   <211> 7
   <212> PRT
   <213> Artificial
<220>
   <223> An artificially synthesized peptide sequence
<400> 18
   Asp Tyr Lys Asp Asp Asp Lys
   1               5

## Description

### Technical Field

The present invention relates to pharmaceuticals that promote functional regeneration of damaged tissues.

### Background Art

Recent years have revealed that various stem cells contribute towards the repairing processes of damaged tissues, and novel regenerative medicines that induce functional tissue regeneration by mobilizing a large number of stem cells to lesion sites are being progressively developed. To realize these novel regenerative medicines, it is necessary that: (i) stem cells that are mobilizable to lesion sites are abundantly present *in vivo*; and (ii) factors that mobilize stem cells to lesion sites have been isolated/identified.

Examples of stem cells that are mobilizable to lesion sites include tissue stem cells existing in lesion areas or nearby tissues, and bone marrow-derived stem cells existing in peripheral blood. In recent years, contribution of bone marrow-derived cells to many types of damaged tissue regenerations has been reported, but the mechanism for mobilizing bone marrow-derived cells to lesion sites is unknown. Bone marrow-derived cells as used herein are distinguished from hematopoietic stem cells which have the potential to differentiate into blood cells (leukocytes and erythrocytes), and include stem cells represented by cells called bone marrow mesenchymal stem cells, or tissue progenitor cell groups exiting in the bone marrow. Bone marrow mesenchymal stem cells are undifferentiated stem cells having the potential to differentiate into osteoblasts, adipocytes, and chondrocytes, and can further differentiate into other mesenchymal cells such as fibroblasts, muscle cells, stromal cells, and tendon cells. Recently, it has been proved that bone marrow mesenchymal stem cells differentiate into nerve cells, and furthermore to epithelial cells (such as skin keratinocytes) and vascular endothelial cells (Non-patent Document 9). Tissue progenitor cells are defined as undifferentiated cells having a unidirectional potential to differentiate into specific tissues/cells other than those of the blood system, and include undifferentiated cells having the potential to differentiate into mesenchymal tissue, epithelial tissue, nerve tissue, parenchymatous organs, and vascular endothelium, as mentioned above.

HMGB1 (High Mobility Group Box 1: High mobility group 1 protein) is a protein having a molecular weight of about 25,000 which exists in almost all kinds of cells *in vivo.* According to previous reports, the following functions are known:
1) HMGB1 regulates gene expression by intracellularly binding with DNA to control chromatin structure (Non-patent Document 1);
2) HMGB1 is secreted from monocytes or macrophages existing in inflammatory tissues by the action of inflammatory cytokines TNF-α, IL-1, and LPS, and extracellularly binds to RAGE (Receptor for Advanced Glycation End products) (Non-patent Document 2) to induce strong inflammatory reactions (Non-patent Document 3);
3) HMGB1 is released from hypoperfusion-induced necrosed cells to the surrounding tissues (Non-patent Document 4);
4) HMGB 1 is associated with inflammation progress in patients with septicemia, a severe infectious disease (Non-patent Document 5);
5) HMGB1 administration to infarcted areas of myocardial infarction models promotes the division/proliferation of stem cells existing in the myocardium so as to promote the regeneration/functional recovery of the myocardium (Patent Document 1);
6) HMGB1 administration to model animals with hypoperfusive liver failure prior to the induction of hypoperfusive conditions alleviates the degree of hepatic impairment (Non-patent Document 6);
7) HMGB1 administration to lesion sites of muscle injury models directs simultaneously-administered vascular progenitor cells to lesion sites so as to promote muscular tissue regeneration (Non-patent Document 7); and
8) HMGB1 induces neurite formation in nerve cells (Non-patent Document 8). However, no previous reports showed that bone marrow-derived stem cells, in particular those mesenchymal stem cells that can differentiate into osteoblasts, chondrocytes, adipocytes, and the like, were mobilized to damaged tissues.

Conventionally, central nerve cells in the brain and the spinal cord were believed to be unregeneratable if once damaged. However, the existence of neural stem cells became known recently and the induction of these cells has become possible. The neural stem cell niche within the normal nerve system has also been identified. Therefore, the recovery of damaged central neurons, which was long considered impossible, is now expected to be feasible. Currently, research related to neuronal regeneration for brain and spinal cord injury, degenerative diseases, and the like are being vigorously developed.

Main causes of brain tissue (cells) injury are traumatic cerebral contusion and cerebral ischemic diseases. Other causes can be injury resulting from brain surgeries such as brain tumor removal. In particular, the total removal of neuroglioma arising from cerebral parenchymal cells is difficult, and there is no choice but to stop at partial removal to avoid damage to motor and language functions. Moreover, malignant neuroglioma has a worse prognosis, and none of the treatments from chemotherapy and radiotherapy to immunotherapy/gene therapy that are actively researched these days, have achieved satisfactory effects. Accordingly, an ideal treatment would be one that can remove as many tumor cells as possible, and restore damage to cerebral functions resulting from the removal.
[Non-patent Document 1] Bustin et al. Mol Cell Biol, 19: 5237-5246, 1999
[Non-patent Document 2] Horiet et al. J. Biol. Chem., 270, 25752-25761, 1995
[Non-patent Document 3] Wang et al. Science, 285: 248-251, 1999
[Non-patent Document 4] Muller et al. EMBO J, 20: 4337-4340, 2001
[Non-patent Document 5] Wang et al. Science, 285: 248-251, 1999
[Non-patent Document 6] Germani et al. J. Leukoc. Biol., 81, 2007, Electronic Journal Version
[Non-patent Document 7] Palumbo et al. J. Cell Biol., 164: 441-449, 2004
[Non-patent Document 8] Merenmies et al. J. Biol. Chem., 266: 16722-16729, 1991
[Non-patent Document 9] Yaojiong et al. Stem cells, 25:2648-1659
[Patent Document 1] Japanese Patent Kohyo Publication No. (JP-A) 2005-537253 (unexamined Japanese national phase publication corresponding to a non-Japanese international publication)

### Disclosure of the Invention

### [Problems to be Solved by the Invention]

An objective of the present invention is to isolate/identify factors mobilizing cells that differentiate into damaged tissues to lesion sites, and to provide inventions utilizing such factors.

### [Means for Solving the Problems]

Once factors that mobilize cells differentiating into damaged tissues are revealed, the administration of such factors to lesion sites allows the mobilization of a large number of cells (which exist in peripheral blood or local tissues) that differentiate into damaged tissues. Thus, the development of novel regenerative medicines which promote functional tissue regeneration is made possible.

The present inventors examined the possibility that bone marrow-derived cells might be mobilized to skin grafts from non-skin tissues in the process of skin graft transplantation into living tissue, thus contributing to skin tissue regeneration. As a result, they revealed the following for the first time in the world.
1) a large amount of bone marrow-derived cells are mobilized to the grafted skin;
2) the mobilized bone marrow-derived cells are differentiated into any of dermal fibroblasts, adipocytes, muscle cells, vascular endothelial cells, and epidermal keratinocytes in the grafted skin, and mobilized bone marrow-derived cells include bone marrow-derived mesenchymal stem cells;
3) the factors which mobilize bone marrow-derived mesenchymal stem cells from peripheral blood to the grafted skin are HMGB1, HMGB2, and HMGB3 released from the necrosed tissue of recipient skin;
4) purified HMGB1, HMGB2, and HMGB3 promote the migration of mesenchymal stem cells isolated and cultured from bone marrow;
5) activators containing HMGB1 which allow the migration of bone marrow mesenchymal stem cells can be conveniently purified from several organ extracts including skin, brain, and heart, by a column chromatography method using a heparin affinity column;
6) activators which allow the migration of bone marrow mesenchymal stem cells can be conveniently extracted from cultured cells; and
7) a heparin-column purified fraction of skin extract mobilizes a large amount of bone marrow-derived cells in case of brain injury.

Based on these findings, the present application provides the following inventions:
[1] a tissue regeneration promoter comprising any one of the following components (a) to (i) for use in regenerative medicine for epithelial or neurological tissue:
   (a) an HMGB1 protein;
   (b) a cell secreting an HMGB1 protein;
   (c) a vector inserted with a DNA encoding an HMGB1 protein;
   (d) an HMGB2 protein;
   (e) a cell secreting an HMGB2 protein;
   (f) a vector inserted with a DNA encoding an HMGB2 protein;
   (g) an HMGB3 protein;
   (h) a cell secreting an HMGB3 protein; and
   (i) a vector inserted with a DNA encoding an HMGB3 protein;
[2] a kit comprising any one of the following components (a) to (i) for use in regenerative medicine for epithelial or neurological tissue:
   (a) an HMGB1 protein;
   (b) a cell secreting an HMGB1 protein;
   (c) a vector inserted with a DNA encoding an HMGB1 protein;
   (d) an HMGB2 protein;
   (e) a cell secreting an HMGB2 protein;
   (f) a vector inserted with a DNA encoding an HMGB2 protein;
   (g) an HMGB3 protein;
   (h) a cell secreting an HMGB3 protein; and
   (i) a vector inserted with a DNA encoding an HMGB3 protein.

### Brief Description of the Drawings

Fig. 1 depicts a method for producing a GFP bone marrow-transplanted mouse.

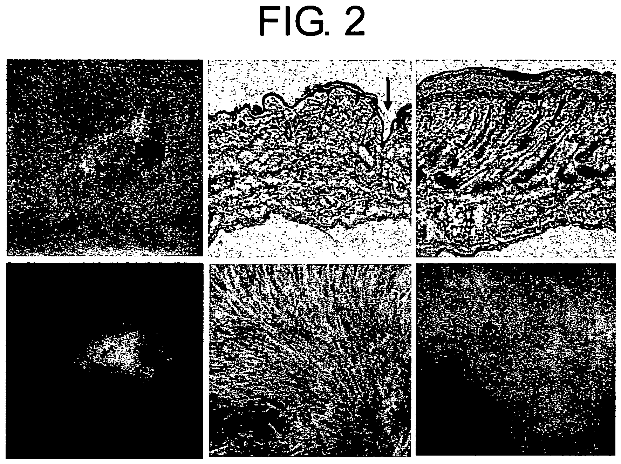

Fig. 2 presents photographs showing the accumulation of GFP fluorescence observed in a skin graft after skin transplantation to the back of a GFP bone marrow-transplanted mouse. Top left is an image of the skin transplantation area seen by the naked eye, top middle is an image of HE-stained tissue of a recipient skin in the vicinity of the boundary between the grafted skin and the recipient skin (shown by the arrow), and top right is an image of HE-stained tissue of the skin graft. Furthermore, bottom left image shows the accumulation of GFP fluorescence in the grafted skin, bottom middle is an enlarged image of the skin transplantation area, and bottom right is an enlarged image showing the accumulation of GFP fluorescence in the same enlarged image of the skin graft.

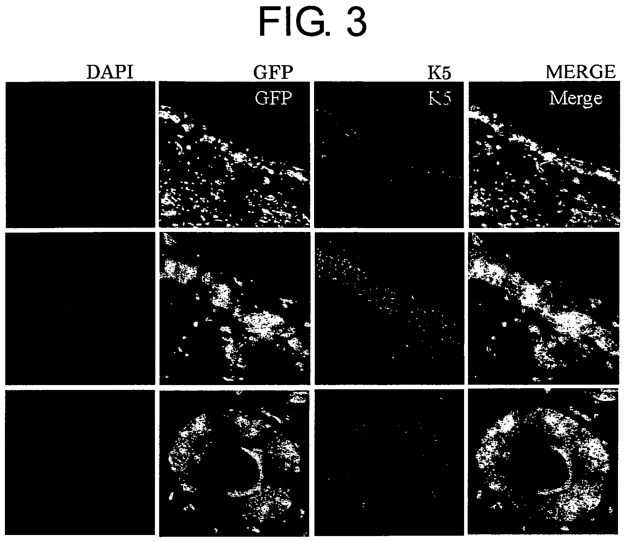

Fig. 3 presents a set of photographs showing bone marrow-derived epidermal cells and bone marrow-derived dermal fibroblasts that accumulated in the grafted skin at the back of the GFP bone marrow-transplanted mouse. The first row on top shows images of the skin of the transplantation area under low magnification (x 100), the middle row shows enlarged images of the same showing the epidermis/dermis boundary under a high magnification (x 200), and the bottom row shows further enlarged images of the same showing a hair follicle under a high magnification (x 200). The far left column shows DAPI staining (nuclear staining), the second column from left shows GFP fluorescence images of the respective regions of the first row. The third column from left shows the immunostaining images of keratin 5 (K5). The fourth column from left shows merged images of each of these fluorescences. Large numbers of GFP-positive epidermal cells and dermal fibroblasts are observed.
Fig. 4 is a flowchart showing a process for identifying a bone marrow-derived mesenchymal stem cell-directing factor in a skin tissue extract.
Fig. 5 depicts a method for extracting a regeneration-inducing factor (bone marrow-derived mesenchymal stem cell-mobilizing factor) from an excised piece of skin.

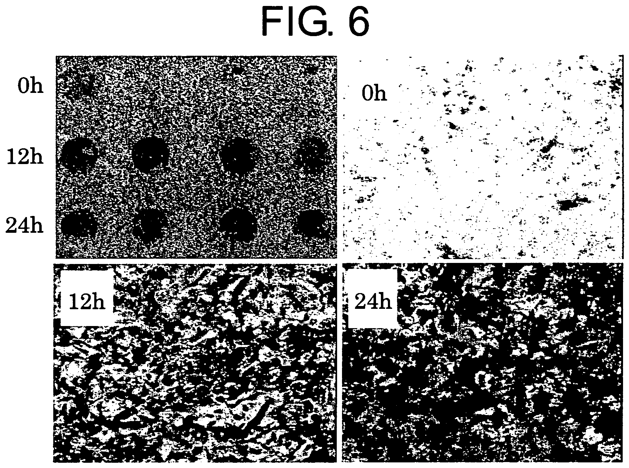

Fig. 6 presents a set of photographs showing assay results of the migratory ability/activity of bone marrow-derived mesenchymal stem cells in a skin extract using a Boyden chamber. The top left picture shows bone marrow mesenchymal stem cells adhered onto a silicone membrane on the lower chamber side, having migrated from the upper chamber of the Boyden chamber to the skin extract side (lower chamber side) through fine pores in the silicone membrane, which are stained with a blue pigment. The stained images are shown immediately after culturing (0 h), after 12 hours (12 h), and after 24 hours (24 h) (four wells each) from the top. The top right picture is an enlarged image (0 h) under a high magnification. Bottom left is an enlarged image (12 h) under a high magnification. Bottom right is an enlarged image (24 h) under a high power magnification.

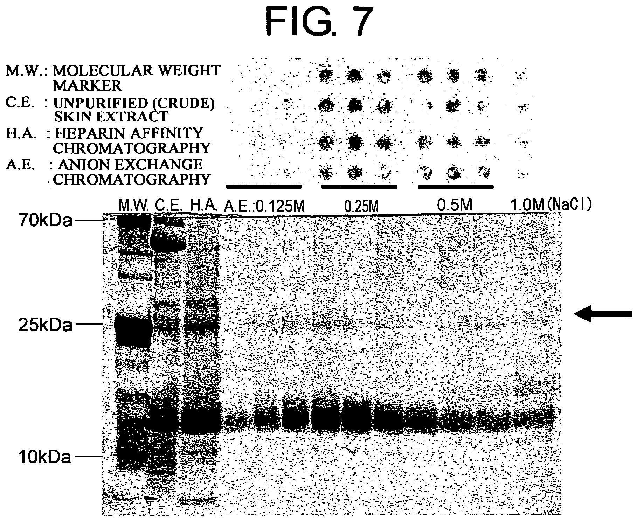

Fig. 7 presents a photograph showing the result of bone marrow-derived mesenchymal stem cells migratory ability/activity assay, examined in the skin extract-purified fraction preparation group using the Boyden chamber, and correspondence with the SDS-PAGE electrophoresis result for each purified fraction preparations. From the left, Lane 1 (M.W.): molecular weight marker; Lane 2 (C.E.): crude skin extract, Lane 3 (H.A.): heparin affinity column-binding fraction (semipurified fraction); and Lanes 4 to 13 (A.E.): anion exchange column-binding fractions (final purified fraction) eluted with various NaCl concentrations, which were all stained with silver after migration. Further, each stained band in the final purified fraction of No. 4 which showed the strongest bone marrow-derived mesenchymal stem cells migration activity by a silver-stained image of the electrophoresis gel (lane 7), were cut out, and then mass spectrometry and database analysis were performed. The result revealed that the band indicated by the arrow is HMGB1.

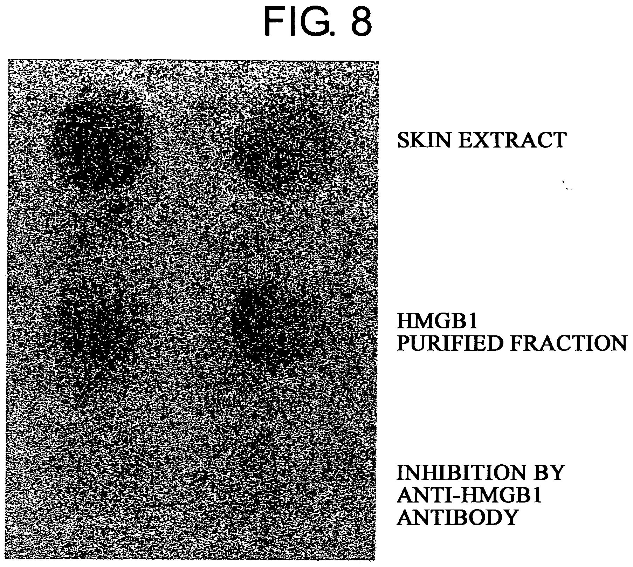

Fig. 8 presents a photograph showing the result of bone marrow-derived mesenchymal stem cell migratory activity assay for HMGB1 using a Boyden chamber. The two images on the top are stained images of bone marrow-derived mesenchymal stem cells, having migrated into the skin extract. The middle two images are stained images of bone marrow-derived mesenchymal stem cells, having migrated into the HMGB1 purified preparation. In the bottom are stained images of bone marrow-derived mesenchymal stem cells (virtual loss of migration activity), having migrated into a neutralized solution obtained by adding an anti-HMGB1 polyclonal antibody to the HMGB1 purified preparation used for the middle.
Fig. 9 shows an *in vivo* assay method for bone marrow-derived mesenchymal stem cell directing activity.
Fig. 10 presents a set of photographs showing the *in vivo* bone marrow-derived mesenchymal stem cell-mobilizing activity of HMGB1. The HMGB1 fraction (final purified fraction No. 4) showed about three times mobilization activity as compared to the comparative control (final purified fraction No. 1).
Fig. 11 presents a photograph showing cells mobilized *in vivo* by the HMGB1 fraction (final purified fraction No. 4) under a high magnification.
Fig. 12 presents a set of photographs showing images immediately after commencing culture of cells that migrated into a silicon tube. On the left is an image of migratory cells inoculated into a medium under a light field, and the right shows a GFP fluorescence image under a dark field.
Fig. 13 presents a set of photographs showing images 24 hours after commencing culture of cells that migrated into the silicon tube. The left picture shows an image of fibroblast-like cells and epithelial-like cells that proliferated and adhered onto the plastic culture dish under a light field, and the right picture shows a GFP fluorescence image under a dark field.

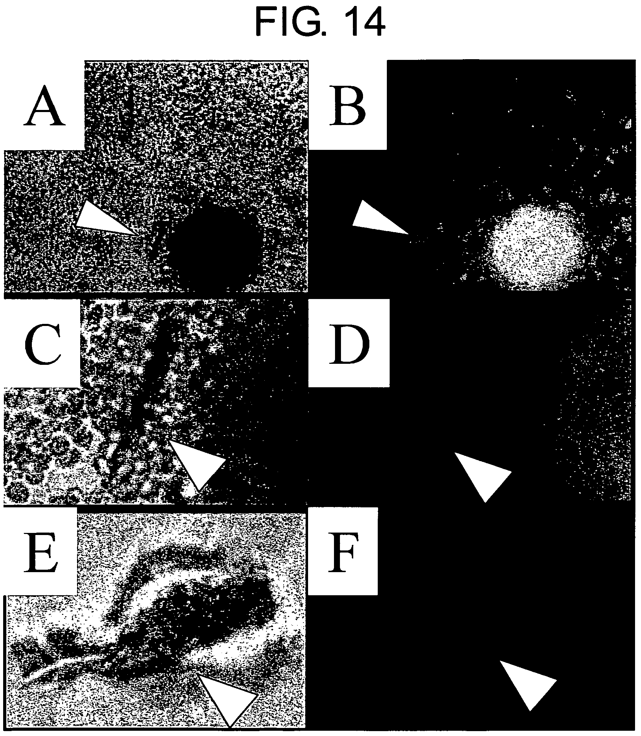

Fig. 14 presents a set of photographs showing images 2 weeks after commencing culture of cells that migrated into the silicon tube. The left and right photographs show the same field of view, in which the left shows images under a light field, whereas the right shows images through a fluorescence filter (GFP fluorescence is detected in B and D and fluorescence of keratin 5 is detected in F). A hair-like linear shape (indicated by the open triangle (arrow)) is observed on the left side of bone marrow-derived GFP-positive cell groups forming circular colonies on the plastic culture dish. F indicates that bone marrow-derived cells are morphologically transformed into a hair-like form, and are further expressing keratin 5 (indicated by the open triangle (arrow)).
Fig. 15 presents a set of photographs showing the HMGB family in a newborn mouse skin extract, detected by the Western blot method.
Fig. 16 shows an illustration of an expression vector map for the HMGB family in mammalian cells, which has, downstream of the promoter, a cytomegalovirus enhancer and a chicken β-actin promoter to synthesize a large amount of mRNAs encoded by the cDNA (complementary DNA) of the HMGB family.
Fig. 17 presents a set of photographs showing the result of Western blotting of the purified recombinant Flag tag-HMGB family-fusion proteins expressed in HEK293 cells.
Fig. 18 presents a set of graphs showing the migration activity of bone marrow mesenchymal stem cells by recombinant HMGB1/HMGB2/HMGB3 using a Boyden chamber. All recombinant proteins showed higher migration activities as compared to the control groups.
Fig. 19 presents a set of graphs showing the result of treatment on mouse cutaneous ulcer treatment models using HMGB family. HMGB1, HMGB2, and HMGB3 all showed significant effects on reducing the ulcer area as compared to control groups.
Fig. 20 presents a photograph which confirms human HMGB1's and a human skin extract's migration-inducing activity on human bone marrow-derived mesenchymal stem cells, using a Boyden chamber.
Fig. 21 presents a set of photographs which confirm activities of bone marrow mesenchymal stem cell-inducing activators in the heart, brain, and skin extracts of the mouse using a Boyden chamber, after the activators were purified by a heparin column.
Fig. 22 presents a set of photographs which confirm human bone marrow mesenchymal stem cell-migrating activities of a cultured cell line HEK293 extract and a HeLa extract, assessed using a Boyden chamber. Both cultured cell lines showed migrating activities on human bone marrow mesenchymal stem cells.

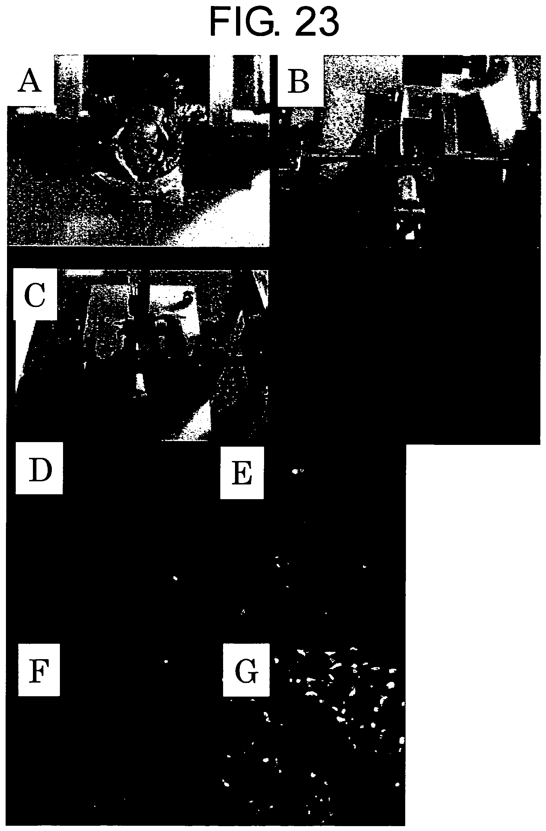

Fig. 23A is a photograph showing a mouse fixed to a brain stereotaxic apparatus and subjected to a midline incision in the head with a scalpel, followed by trepanation using a drill. Fig. 23B is a photograph showing the brain to which a negative pressure is applied using a syringe to suck a part of the brain tissue. Fig. 23C is a photograph after injection of 5 µl heparin-column purified fraction of a skin extract dissolved in fibrin adhesive formulation (fibrinogen) to the brain, and a subsequent injection of 5 µl of fibrin glue formulation (thrombin). Fig. 23D and Fig. 23E are photographs of the brain injury model taken 2 weeks after the treatment. Higher accumulation of GFP-positive cells was observed in the treatment group using the heparin-column purified fraction of skin extract in 23E compared to the control in 23D. Fig. 23F and Fig. 23G are photographs of the brain injury model taken 6 weeks after the treatment. Higher accumulation of GFP-positive cells was observed in the treatment group using the heparin-column purified fraction of skin extract in 23G compared to the control in 23F.

### Best Mode for Carrying Out the Invention

Disclosed are inducers of bone marrow-derived cells, comprising at least one of the following ingredients (a) to (i):
(a) an HMGB1 protein;
(b) a cell secreting an HMGB1 protein;
(c) a vector inserted with a DNA which encodes an HMGB 1 protein;
(d) an HMGB2 protein;
(e) a cell secreting an HMGB2 protein;
(f) a vector inserted with a DNA which encodes an HMGB2 protein;
(g) an HMGB3 protein;
(h) a cell secreting an HMGB3 protein; and
(i) a vector inserted with a DNA which encodes an HMGB3 protein.

With use of such an inducer, bone marrow-derived cells (such as bone marrow-derived mesenchymal stem cells) are directed to local areas (area of administration/addition with the above ingredient, or the vicinity thereof), enabling the promotion of functional tissue regeneration. Therefore, an inducer can be used as a reagent necessary for basic and clinical research for the development of regenerative medicine and regeneration inducing medicine. For example, it becomes possible to mobilize bone marrow-derived cells (such as bone marrow-derived mesenchymal stem cells) to a required tissue *in vivo* in an experimental animal so as to examine the degree of tissue repairment and the reconstruction of tissue function. *Moreover, in vitro* research on tissue regeneration induction by mobilizing bone marrow-derived cells (such as bone marrow-derived mesenchymal stem cells) can be performed.

Further, regeneration of the damaged tissue can be promoted by using an inducer. Use of an above inducer as a so-called preventive drug which prevents deteriorations in tissue/organ functions caused by the reduction of tissue stem cells, or as an anti-aging drug which delays the progress of age-related alterations are anticipated besides the use as an inducer/promoter of functional tissue regeneration.

The present invention also provides tissue regeneration promoters, or kits comprising at least one of the following ingredients (a) to (i) for use in regenerative medicine for epithelial or neurological tissue:
(a) an HMGB1 protein;
(b) a cell secreting an HMGB1 protein;
(c) a vector inserted with a DNA which encodes an HMGB 1 protein;
(d) an HMGB2 protein;
(e) a cell secreting an HMGB2 protein;
(f) a vector inserted with a DNA which encodes an HMGB2 protein;
(g) an HMGB3 protein;
(h) a cell secreting an HMGB3 protein; and
(i) a vector inserted with a DNA which encodes an HMGB3 protein.

The tissue regeneration promoters or kits for use according to the present invention are characterized in that administration to a damaged tissue area or the vicinity directs bone marrow-derived cells (such as bone marrow-derived mesenchymal stem cells) circulating in blood, from the peripheral blood to the damaged tissue (local induction).

Disclosed are methods for producing an extract of cells having an activity of inducing bone marrow-derived cells, which comprise the step of immersing the cells in a solvent. Moreover, disclosed are extracts of cells produced by the above production method and which have an activity of inducing bone marrow-derived cells.

Cells to be immersed in a solvent are not specifically limited, and examples include tissue-derived cells and cell lines established from tissue-derived cells (such as Hela and HEK293, but not limited thereto), isolated cells, non-isolated cells (such as cells existing in isolated tissues), cells introduced with a DNA encoding an HMGB1, HMGB2, or HMGB3 protein. Any tissue may be used as the tissue described as above. For example, such tissues include, but are not limited to, living skin tissues or tissues obtained from internal biopsies (operations) (such as brain, lung, heart, liver, stomach, small intestine, large intestine, pancreas, kidney, bladder, spleen, uterus, testis, and blood).

Examples of the above solvent include, but are not limited to, physiological saline, PBS (phosphate-buffered saline), and TBS (Tris-buffered saline). Moreover, the immersion time of cells or tissue in a solvent should be a duration necessary and sufficient for inducing cell necrosis, that is, 1 hour to 48 hours (such as 6 to 48 hours), and preferably 12 to 24 hours, but is not limited thereto. Therefore, the "step of immersing cells in a solvent" can be rephrased as "step of immersing cells in a solvent for a duration necessary and sufficient for inducing necrosis" or "step of necrosing cells". Moreover, examples of the temperature for immersing cells or tissue in a solvent include, but are not limited to, 4°C to 25°C (such as 4°C to 8°C), and preferably 4°C. Further, examples of the pH for immersing cells or tissue in a solvent include, without limitation, pH 7 to 8, and preferably pH 7.5. Examples of the buffer include, without limitation, a phosphate buffer solution at a concentration of 10 mM to 50 mM, preferably 10 to 20 mM.

Moreover, cells or tissues can be removed from a solvent containing them after immersing them in the solvent. The method for removing cells or tissues from a solvent is not particularly limited as long as the method is well known to those skilled in the art. For example, cells or tissues can be removed from a solvent by centrifuging at a gravity acceleration of 10 G to 4000 G (for example, 440 G) at 4°C to 25°C (for example, 4°C), followed by separating the supernatant, but the removal method is not limited thereto. The supernatant can be used as an extract of cells or tissues.

Further, disclosed are methods for producing a heparin-binding fraction having an activity of inducing bone marrow-derived cells, which comprise the following steps. Moreover, disclosed are heparin-binding fractions produced by the above production method, which have an activity of inducing bone marrow-derived cells.
(a) a step of immersing the cells or tissue in a solvent;
(b) a step of contacting an extract obtained by the step (a) with immobilized heparin; and
(c) a step of eluting a heparin-binding fraction (may also be expressed as heparin-purified fraction or heparin-column purified fraction) from the immobilized heparin.

"Immobilized heparin" refers to heparin which is covalently bound to an insoluble carrier. Examples of the insoluble carrier include, but are not limited to, Sepharose beads (such as Sepharose 4B, Sepharose 6B and such: GE Healthcare). A commercially available immobilized heparin (Hitrap Heparin HP column: GE Healthcare) may also be used.

Examples of conditions for contacting an extract of cells or tissues with immobilized heparin include, but are not limited to, about pH 7 to 8 (preferably pH 7.5), and a salt concentration of 0 to 200 mM, and preferably about 100 to 200 mM. The time the extract is in contact with immobilized heparin is not specifically limited, but the contact is preferably retained for 5 minutes or more in view of a sufficient absorption of the heparin-binding fraction onto immobilized heparin. Examples of the temperature include, but are not limited to, 4 to 8°C, and preferably 4°C. Further, examples of the elution condition of the heparin-binding fraction absorbed onto the immobilized heparin include, but are not limited to, a pH of about 7 to 8 and a salt concentration of 200 to 1000 mM (preferably about 1000 mM).

Further, disclosed are methods for producing an anion exchanger-binding fraction having an activity of inducing bone marrow-derived cells, which comprise the following steps.
(a) a step of immersing cells or tissues in a solvent;
(b) a step of contacting an extract obtained by step (a) with immobilized heparin;
(c) a step of eluting a heparin-binding fraction from the immobilized heparin;
(d) a step of contacting the heparin-binding fraction obtained by step (c) with an anion exchanger; and
(e) a step of eluting an anion exchanger-binding fraction from the anion exchanger.

Moreover, disclosed are anion exchanger-binding fractions produced by the above production method and having an activity of inducing bone marrow-derived cells.

Examples of the anion exchanger include, but are not limited to, exchangers using DEAE (diethylaminoethyl) or Q (quaternary ammonium). Commercially available anion exchangers (such as Source15Q Source30Q, MonoQ, MiniQ, PC3.2/3, Mini Q4.6/50 PE, HiTrap IEX Columns, HiTrap SP HP, Q Sepharose High Performance, Hiload 16/10 Q Sepharose HP, HiPrep 16/10 SP XL, Q Sepharose XL, HiPrer 16/10 Q FF, HiPrep 16/10DEAE FF, Q Sepharose Fast Flow, and DEAE Sepharose Fast Flow (all from GE Healthcare)) may also be used.

Examples of the conditions for contacting a heparin-binding fraction with an anion exchanger are a pH of about 7 to 9 (preferably pH 8), and a salt concentration of 0 to 100 mM, preferably about 50 mM, but are not limited thereto. The time for contacting an extract with an anion exchanger is not particularly limited, but the contact is preferably retained for 5 minutes or more in view of a sufficient absorption of the anion exchanger-binding fraction onto the anion exchanger. Examples of the temperature include, but are not limited to, 4 to 16°C, and preferably 4°C. Further, examples of conditions for eluting the anion exchanger-binding fraction absorbed onto the anion exchanger include, but are not limited to, a pH of about 7 to 9 (preferably about pH 8) and a salt concentration of 100 to 2000 mM (preferably about 1000 mM).

Disclosed are inducers of bone marrow-derived cells, comprising the above-mentioned extract, the above-mentioned fraction, or an extract or fraction produced by the above-mentioned method.

With the use of such an inducer, bone marrow-derived cells (such as bone marrow-derived mesenchymal stem cells) are directed to local areas (area to which the above ingredient is administered/added, or an area in the vicinity thereof) which allows the promotion of functional tissue regeneration. Therefore, the inducer can be used as a reagent necessary for basic and clinical research for the development of regenerative medicines and regeneration-inducing medicines. For example, it becomes possible to mobilize bone marrow-derived cells (such as bone marrow-derived mesenchymal stem cells) to a required *tissue in vivo* in an experimental animal so as to examine the degree of tissue repairment and reconstruction of tissue function. Moreover, *in vitro* research on tissue regeneration induction by mobilizing bone marrow-derived cells (such as bone marrow-derived mesenchymal stem cells) can be performed.

Further, damaged tissue regeneration can be promoted by using the above inducer. Besides the use as an inducer/promoter of functional tissue regeneration, the above inducer is also anticipated to be used as a so-called preventive drug that prevents tissue/organ function deteriorations caused by reduction of tissue stem cells, or as an anti-aging drug which delays the progress of age-related alterations.

Bone marrow-derived cells (such as bone marrow-derived mesenchymal stem cells) that are mobilized to the damaged tissue differentiate into various types of cells to contribute to functional regeneration of the damaged tissue and maintenance/enhancement of the functions. In the present invention, examples of damaged tissue include, but are not limited to, tissues damaged by various pathological conditions, trauma, burns, inflammation, autoimmunity, gene abnormalities, and the like causing ischemic/hypoperfusive/hypoxic conditions. Damaged tissue also includes necrosed tissues.

Tissues in the present invention are epithelial and neurological tissues. Moreover, with use of the above tissue regeneration promoters, treatments for inducing functional tissue regeneration becomes possible not only in cutaneous diseases such as intractable cutaneous ulcers, skin wounds, bullosis, and alopecia, but also in tissue damages such as cerebral infarction, myocardial infarction, bone fracture, pulmonary infarction, gastric ulcers, and enteritis. The types of animals to be administered with the above tissue regeneration promoter include human and non-human animals, which can be exemplified by, but are not limited to, humans, mice, rats, monkeys, pigs, dogs, rabbits, hamsters, and guinea pigs.

Bone marrow-derived cells of the present disclosure are cells other than hematopoietic stem cells, or cells derived therefrom such as leukocytes, erythrocytes, and platelets, and include stem cells represented by cells which have been hitherto called bone marrow mesenchymal stem cells and tissue progenitor cell populations existing in the bone marrow. Bone marrow-derived cells of the present disclosure can be isolated from bone marrow blood collection or peripheral blood collection. Hematopoietic stem cells are nonadherent, while bone marrow-derived cells of the present disclosure are obtained as adherent cells by means of a cell culture of a monocyte fraction of blood obtained from the bone marrow blood collection or peripheral blood collection. Moreover, bone marrow-derived cells of the present disclosure include mesenchymal stem cells, and have a potential to differentiate into, preferably, osteoblasts (the induction of differentiation can be identified by observing calcification), chondrocytes (which can be identified by alcian blue positive staining, safranin O positive staining, or the like), adipocytes (which can be identified by Sudan III positive staining), and other mesenchymal cells such as fibroblasts, smooth muscle cells, stromal cells, and tendon cells; and further nerve cells, epithelial cells (for example, epidermal keratinocytes and intestinal epithelial cells express cytokeratin family), and vascular endothelial cells. However, the cells to be differentiated into are not limited to the above cells, and the potential to differentiate into cells of parenchymatous organs such as liver, kidney, and pancreas are also included.

In the present disclosure, bone marrow-derived mesenchymal stem cells refer to cells existing in the bone marrow, which are directly collected from the bone marrow or indirectly collected from other tissues (mesenchymal tissues such as blood, skin, and fat), and can be cultured and proliferated as adherent cells on a culture dish (made of plastic or glass). These cells are characterized in having a potential to differentiate into mesenchymal tissues such as bone, cartilage, and fat, and can be obtained from a collection of bone marrow blood, peripheral blood, or mesenchymal tissues. Bone marrow-derived mesenchymal stem cells are also characterized in having a potential to differentiate into epithelial tissues such as keratinocytes that constitute skin, by administrating these cells that have once adhered onto a culture dish to a lesion area of the living body.

Bone marrow-derived mesenchymal stem cells of the present disclosure are multipotent stem cells, and have a potency to differentiate preferably into: osteoblasts (the induction of differentiation can be identified by observing calcification), chondrocytes (which can be identified by alcian blue positive staining, safranin O positive staining, or the like), adipocytes (which can be identified by Sudan III positive staining), and other mesenchymal cells such as fibroblasts, smooth muscle cells, skeletal muscle cells, stromal cells, and tendon cells; nerve cells, pigment cells, epidermal cells, hair follicle cells (which express cytokeratin family, hair keratin family, or the like), epithelial cells (for example, epidermal keratinocytes and intestinal epithelial cells express cytokeratin family or the like), and endothelial cells; and further preferably into cells of parenchymatous organs such as liver, kidney, and pancreas. However, differentiated cells are not limited to the above cells.

Moreover, human bone marrow mesenchymal stem cells can be exemplified by, but are not limited to, cells which can be directly obtained from collecting bone marrow blood, peripheral blood, or fat, or obtained as adherent cells through culturing of an isolated monocyte fraction. Markers for human bone marrow mesenchymal stem cells can be, for example, all or some of the markers of Lin-negative, CD45-negative, and CD44-positive, but are not limited to.

Moreover, mouse bone marrow mesenchymal stem cells can be exemplified by, but are not limited to, cells which can be obtained by methods described in the Examples. Markers for mouse bone marrow mesenchymal stem cells can be for example, all or some of the markers of Lin-negative, CD45-negative, CD44-positive, Sca-1 positive, and c-kit negative, but are not limited to.

Tissue progenitor cells are defined as undifferentiated cells having a unidirectional potency to differentiate into specific tissues/cells other than the blood system, and include undifferentiated cells having the potency to differentiate into mesenchymal tissue, epithelial tissue, nerve tissue, parenchymatous organs, and vascular endothelium as mentioned above.

For tissue regeneration promoters for use according to the present invention, there is no particular limitation in components other than the at least one of the ingredients (a) to (i) mentioned above, so long as the component does not inhibit the induction of bone marrow-derived cells (such as bone marrow-derived mesenchymal stem cells) and the promotion of tissue regeneration. For example, in addition to the at least one of the substances (a) to (i) mentioned above, the tissue regeneration promoters for use according to the present invention may contain: molecules (molecular groups) related to the enhancement of the function of HMGB1, HMGB2, or HMGB3 to induce functional tissue regeneration; molecules (molecular groups) which inhibit unanticipated actions of HMGB1, HMGB2, or HMGB3; factors which regulate proliferation and differentiation of bone marrow-derived cells (such as bone marrow-derived mesenchymal stem cells); and other factors which enhance/maintain these factors or cellular functions.

The types of animals which serve as a source of HMGB1, HMGB2, or HMGB3 protein for the tissue regeneration promoters for use according to the present invention include human and non-human animals, which can be exemplified by humans, mice, rats, monkeys, pigs, dogs, rabbits, hamsters, and guinea pigs, but the kind is preferably the same as the animal to be administered with the HMGB1, HMGB2, or HMGB3 protein.

HMGB1 proteins in tissue regeneration promoters for use according to the present invention can be exemplified by, but are not limited to, proteins comprising the amino acid sequence of SEQ ID NO: 1, 3, or 5. HMGB1 proteins employed according to the present invention can also include proteins which are functionally equivalent to the protein comprising the amino acid sequence of SEQ ID NO: 1, 3, or 5. Examples of such proteins include: 1) isolated proteins which comprise an amino acid sequence with one or more amino acid substitutions, deletions, insertions, and/or additions in the amino acid sequence of SEQ ID NO: 1, 3, or 5, and which are functionally equivalent to the protein comprising the amino acid sequence of SEQ ID NO: 1, 3, or 5; and 2) isolated proteins which are encoded by DNAs that hybridize under stringent conditions with DNAs comprising the nucleotide sequence of SEQ ID NO: 2, 4, or 6, and which are functionally equivalent to the protein comprising the amino acid sequence of SEQ ID NO: 1, 3, or 5.

HMGB2 proteins in tissue regeneration promoters for use according to the present invention can be exemplified by, but are not limited to, proteins comprising the amino acid sequence of SEQ ID NO: 7, 9, or 11. HMGB2 proteins employed according to the present invention can also include proteins which are functionally equivalent to the protein comprising the amino acid sequence of SEQ ID NO: 7, 9, or 11. Examples of such proteins include: 1) isolated proteins which comprise an amino acid sequence with one or more amino acid substitutions, deletions, insertions, and/or additions in the amino acid sequence of SEQ ID NO: 7, 9, or 11, and which are functionally equivalent to the protein comprising the amino acid sequence of SEQ ID NO: 7, 9, or 11; and 2) isolated proteins which are encoded by DNAs that hybridize under stringent conditions with DNAs comprising the nucleotide sequence of SEQ ID NO: 8, 10, or 12, and which are functionally equivalent to the protein comprising the amino acid sequence of SEQ ID NO: 7, 9, or 11.

HMGB3 proteins in tissue regeneration promoters for use according to the present invention can be exemplified by, but are not limited to, proteins comprising the amino acid sequence of SEQ ID NO: 13 or 15. HMGB3 proteins employed according to the present invention can also include proteins which are functionally equivalent to the protein comprising the amino acid sequence of SEQ ID NO: 13 or 15. Examples of such proteins include: 1) isolated proteins which comprise an amino acid sequence with one or more amino acid substitutions, deletions, insertions, and/or additions in the amino acid sequence of SEQ ID NO: 13 or 15, and which are functionally equivalent to the protein comprising the amino acid sequence of SEQ ID NO: 13 or 15; and 2) isolated proteins which are encoded by DNAs that hybridize under stringent conditions with DNAs comprising the nucleotide sequence of SEQ ID NO: 14 or 16, and which are functionally equivalent to the protein comprising the amino acid sequence of SEQ ID NO: 13 or 15.

Isolated proteins which are functionally equivalent to the protein comprising the amino acid sequence of SEQ ID NO: 1, 3, 5, 7, 9, 11, 13 or 15 may be homologues or paralogues to the protein comprising the amino acid sequence of SEQ ID NO: 1, 3, 5, 7, 9, 11, 13 or 15. Those skilled in the art can isolate proteins which are functionally equivalent to the protein comprising the amino acid sequence of SEQ ID NO: 1, 3, 5, 7, 9, 11, 13 or 15, by known methods (supplementary volume of "Jikken Igaku (Experimental Medicine), Idenshi Kougaku Handbook (Genetic Engineering Handbook)", pp246-251, published by Yodosha Co., Ltd., 1991).

Examples of proteins which are functionally equivalent to the protein comprising the amino acid sequence of SEQ ID NO: 1, 3, 5, 7, 9, 11, 13 or 15 include proteins having bone marrow-derived cell (such as bone marrow-derived mesenchymal stem cells)-inducing activity.

Proteins which comprise an amino acid sequence with one or more amino acid substitutions, deletions, insertions, and/or additions in the amino acid sequence of SEQ ID NO: 1, 3, 5, 7, 9, 11, 13 or 15, and which are functionally equivalent to the protein comprising the amino acid sequence of SEQ ID NO: 1, 3, 5, 7, 9, 11, 13 or 15 include naturally-occurring proteins. Generally, eukaryotic genes have a polymorphism as known in interferon genes and such. Alterations in nucleotide sequences caused by the polymorphism, may result in one or more amino acid substitutions, deletions, insertions, and/or additions. Naturally-occurring proteins such as those comprising an amino acid sequence with one or more amino acid substitutions, deletions, insertions, and/or additions in the amino acid sequence of SEQ ID NO: 1, 3, 5, 7, 9, 11, 13 or 15, and which are functionally equivalent to the protein comprising the amino acid sequence of SEQ ID NO: 1, 3, 5, 7, 9, 11, 13 or 15 are included in HMGB1, HMGB2, or HMGB3 proteins of the present invention.

The present invention also includes artificially-produced mutant proteins so long as they are functionally equivalent to the protein comprising the amino acid sequence of SEQ ID NO: 1, 3, 5, 7, 9, 11, 13 or 15. Known methods which cause random mutations to a given nucleotide sequence include substitution(s) of base pair(s) through nitrous acid treatment of DNA (Hirose, S. et al., Proc. Natl. Acad. Sci. USA., 79:7258-7260, 1982). This method enables random introduction of substitution(s) of base pair(s) into a specific segment by nitrous acid treatment of the segment desired to be mutated. Alternatively, technologies for site-directing a target mutation include the gapped duplex method (Kramer W. and Fritz HJ., Methods in Enzymol., 154:350-367, 1987) and the like. A cyclic double stranded vector in which a gene to be introduced with a mutation is cloned, is separated into single strands. These single strands are hybridized with a synthetic oligonucleotide mutated at the target site. A vector-derived complementary single strand DNA linearized by a restriction enzyme is annealed with the cyclic single stranded vector, and the gap between the oligonucleotide and the vector is filled by using a DNA polymerase, which is then made into a complete double stranded vector by ligation.

The number of amino acids to be modified would be typically within 50, preferably within 30, and more preferably within 5 amino acids (for example, one amino acid).

When an amino acid is artificially substituted, substitution with an amino acid having similar properties would result in maintaining the activity of the original protein. Proteins employed according to the present invention include proteins resulting from a conservative substitution in the above substitution of amino acid(s), and which are functionally equivalent to the protein comprising the amino acid sequence of SEQ ID NO: 1, 3, 5, 7, 9, 11, 13 or 15. Conservative substitution is considered important when substituting amino acid(s) of domains important for protein activities. Such a conservative substitution of amino acid(s) is well known to those skilled in the art.

Examples of amino acid groups suitable for conservative substitution include basic amino acids (such as lysine, arginine, and histidine), acidic amino acids (such as aspartic acid and glutamic acid), uncharged polar amino acids (such as glycine, asparagine, glutamine, serine, threonine, tyrosine, and cysteine), nonpolar amino acids (such as alanine, valine, leucine, isoleucine, proline, phenylalanine, methionine, and tryptophane), β branched amino acids (such as threonine, valine, and isoleucine), and aromatic amino acids (such as tyrosine, phenylalanine, tryptophane, and histidine).

Moreover, non-conservative substitution may increase protein activities (for example, constitutively activated proteins).

In addition, proteins which are functionally equivalent to the protein comprising the amino acid sequence of SEQ ID NO: 1, 3, 5, 7, 9, 11, 13 or 15 can be obtained by methods that utilize hybridization. That is to say, a DNA encoding HMGB1, HMGB2, or HMGB3 protein of the present invention as shown in the SEQ ID NO: 2, 4, 6, 8, 10, 12, 14, or 16, or a fragment thereof is used as a probe, and then DNAs that can hybridize to them are isolated. A hybridization reaction performed under stringent conditions leads to the selection of highly homologous DNA as a nucleotide sequence. This increases the chances of isolated proteins containing proteins that are functionally equivalent to the HMGB1, HMGB2, or HMGB3 protein. Examples of a highly homologous nucleotide sequence include those having 70% or more, and desirably 90% or more identity.

In a specific example, the term "stringent conditions" refers to hybridization conditions with 6 x SSC, 40% formamide at 25°C and subsequent washing with 1x SSC at 55°C. The stringency depends on conditions such as salt concentration, formamide concentration, or temperature; however it is obvious for those skilled in the art to set these conditions so as to obtain a necessary stringency.

With the use of hybridization, for example, DNAs encoding homologues of the HMGB1, HMGB2, or HMGB3 proteins other than those proteins comprising the amino acid sequence of SEQ ID NO: 1, 3, 5, 7, 9, 11, 13 or 15, can be isolated.

Proteins which are functionally equivalent to a protein comprising the amino acid sequence of SEQ ID NO: 1, 3, 5, 7, 9, 11, 13 or 15 normally have a high homology with the amino acid sequence of SEQ ID NO: 1, 3, 5, 7, 9, 11, 13 or 15. The term "high homology" refers to a sequence identity of at least 30% or more, preferably 50% or more, more preferably 80% or more (for example, 95% or more). The identity of the nucleotide sequences and amino acid sequences can be determined using a homology search site via the internet (For example, homology searches such as FASTA, BLAST, PSI-BLAST, and SSEARCH can be used in the DNA Data Bank of Japan (DDBJ) [examples of which include the homology search page (Search and Analysis) at the DNA Data Bank of Japan (DDBJ) website; http://www.ddbj.nig.ac.jp/E-mail/homology-j.html]. Furthermore, searches using BLAST can be carried out through the web site of the National Center for Biotechnology Information (NCBI) (examples of which include BLAST page at the homepage of NCBI website; http://www.ncbi.nlm.nih.govBLAST/; Altschul, S.F. et al., J. Mol. Biol., 1990, 215(3):403-10; Altschul, S.F. & Gish, W., Meth. Enzymol., 1996, 266:460-480; Altschul, S.F. et al., Nucleic Acids Res., 1997, 25:3389-3402)).

For example, in the calculation of the identity of amino acid sequences using Advanced BLAST 2.1, the identity value (%) can be obtained by the following: blastp is used as the program, expect value is set at 10, all filters are set at OFF, BLOSUM62 is used for matrix, and gap existence cost, per residue gap cost, and lambda ratio are set at 11, 1, and 0.85, respectively (default parameters) (Karlin, S. and S. F. Altschul (1990) Proc. Natl. Acad. Sci. USA 87:2264-68; Karlin, S. and S. F. Altschul (1993) Proc. Natl. Acad. Sci. USA 90:5873-7).

Proteins employed according to the present invention, or proteins functionally equivalent thereto may be proteins subjected to various modifications such as physiological modification with sugar chains and the like, labeling with fluorescence or radioactive substances, or fusion with other proteins. Particularly in recombinants that will be described later, sugar chain modification may vary depending on the hosts used for expression. However, even if there is a difference in sugar chain modifications, all proteins having properties similar to those of HMGB1, HMGB2, or HMGB3 proteins disclosed herein are HMGB1, HMGB2, or HMGB3 proteins of the present invention or proteins functionally equivalent thereto.

HMGB1, HMGB2, or HMGB3 proteins can be obtained not only from living materials, but also in the form of recombinants by incorporating genes that encode these proteins into an appropriate expression system. In order to obtain HMGB1, HMGB2, or HMGB3 proteins by genetic engineering techniques, the above-mentioned DNAs which encode HMGB1, HMGB2, or HMGB3 proteins may be incorporated into an appropriate expression system, and they can then be expressed. Examples of host/vector systems applicable to the present invention include the expression vector pGEX and *E. coli.* With pGEX, foreign genes can be expressed as a fusion protein with glutathione-S-transferase (GST) (Gene, 67:31-40, 1988). pGEX incorporated with a gene encoding HMGB1, HMGB2, or HMGB3 protein is introduced into an *E. coli* strain such as BL21 by heat shock, incubated for an appropriate time and then isopropylthio-β-D-galactoside (IPTG) is added to induce the expression of GST-fused HMGB1, GST-fused HMGB2, or GST-fused HMGB3 proteins. Since GST of the present invention absorbs onto Glutathione Sepharose 4B, the expression product is readily separated and purified by affinity column chromatography.

In addition, the following may also be applied as host/vector systems to obtain recombinants of HMGB1, HMGB2, or HMGB3 proteins. First, when bacteria are used as hosts, expression vectors for fusion proteins that utilize histidine tag, HA tag, a FLAG tag, and the like are commercially available. Regarding yeasts, yeasts belonging to the genus *Pichia* are known to be effective for the expression of sugar chain-containing proteins. In terms of the addition of sugar chains, expression systems that utilize baculovirus vector with insect cells as a host are also useful (Bio/Technology, 6:47-55, 1988). Further, using mammalian cells, transfection of a vector is carried out using promoters such as CMV, RSV, and SV40. Any of these host/vector systems can be used as an expression system of HMGB1, HMGB2, or HMGB3 proteins. Moreover, genes can also be introduced using viral vectors such as retrovirus vectors, adenovirus vectors, and adeno-associated virus vectors.

Thus obtained proteins employed according to the present invention may be isolated intracellularly or extracellularly (medium and such), and can be purified as proteins that are substantially pure and homogenous. Proteins may be separated and purified using separation and purification methods which are commonly used in protein purification, and are not particularly limited. For example, proteins can be separated and purified by appropriately selecting and combining a chromatography column, filter, ultrafiltration, salting out, solvent precipitation, solvent extraction, distillation, immunoprecipitation, SDS-polyacrylamide gel electrophoresis, isoelectric focusing electrophoresis, dialysis, recrystallization, and the like.

Examples of chromatographies include affinity chromatography, ion exchange chromatography, hydrophobic chromatography, gel filtration, reverse phase chromatography, and adsorption chromatography (Marshak et al., Strategies for Protein Purification and Characterization: A Laboratory Course Manual. Ed Daniel R. Cold Spring Harbor Laboratory Press, 1996). These chromatographies can be performed using liquid phase chromatographies such as HPLC and FPLC.

Moreover, proteins employed according to the present invention are preferably substantially purified proteins. Here, the term "substantially purified" means that the protein purity of the present invention (proportion of the protein of the present invention in total protein components) is 50% or more, 60% or more, 70% or more, 80% or more, 90% or more, 95% or more, 100% or close to 100%. The upper limit for "close to 100%" depends on the purification techniques and analytical techniques of those skilled in the art, of which examples are 99.999%, 99.99%, 99.9%, 99%, and the like.

Moreover, a substantially purified protein includes any protein purified by any purification method as long as the protein purity is as mentioned above. Examples include, but are not limited to, proteins substantially purified by appropriately selecting and combining the above-mentioned chromatography column, filter, ultrafiltration, salting out, solvent precipitation, solvent extraction, distillation, immunoprecipitation, SDS-polyacrylamide gel electrophoresis, isoelectric focusing electrophoresis, dialysis, recrystallization, and the like.

Cells where HMGB1, HMGB2, or HMGB3 proteins of the tissue regeneration promoters for use according to the present invention are released or secreted basically include all types of tissue-derived cells *in vivo*. Cells which can be readily collected and cultured are exemplified by, but are not limited to, fibroblasts (such as normal skin fibroblasts and cell lines derived therefrom). Moreover, cells secreting HMGB1, HMGB2, or HMGB3 proteins can also be produced by the following manner. A vector is produced by inserting an HMGB1, HMGB2, or HMGB3 protein-encoding DNA, or an HMGB1, HMGB2, or HMGB3 protein-encoding DNA linked with a secretion signal-encoding DNA (ATG CAG ACA GAC ACA CTC CTG CTA TGG GTA CTG CTG CTG TGG GTT CCA GGT TCC ACT GGT GAC; SEQ ID NO: 17), into a known expression vector or a gene therapy vector. The produced vector is introduced into mammalian cells such as fibroblasts (such as normal skin fibroblasts and cell lines derived therefrom), insect cells, and other cells. There are no particular limitations in the animal types from which these cells derive, although cells from the animal type of the target animal subjected to tissue regeneration, cells from the target itself, or cells derived from a blood relative of the target subjected to tissue regeneration are preferably used.

DNAs which encode HMGB1, HMGB2, or HMGB3 proteins of the tissue regeneration promoters for use according to the present invention may be cDNAs, genomic DNAs, natural DNAs, or artificially-synthesized DNAs so long as they encode the HMGB1, HMGB2, or HMGB3 protein. DNAs which encode HMGB1, HMGB2, or HMGB3 proteins are normally contained in tissue regeneration promoters for use according to the present invention in a form inserted in vectors (such as gene therapy vectors).

Examples of the gene therapy vectors of the present invention include, but are not limited to, plasmid vectors, retrovirus vectors, lentivirus vectors, adenovirus vectors, adeno-associated virus vectors, Sendai virus vectors, Sendai virus envelope vectors, and papilloma virus vectors. The gene therapy vectors may contain promoter DNA sequences which effectively induce gene expression, factors that regulate gene expression, and molecules which are necessary for maintaining DNA stability.

Tissue regeneration promoters for use according to the present invention may also contain: partial peptides of HMGB1, HMGB2, or HMGB3 protein which have an activity of inducing bone marrow-derived cells (such as bone marrow-derived mesenchymal stem cells); cells secreting these partial peptides; or vectors inserted with the DNAs encoding these partial peptides.

Methods for administering tissue regeneration promoters for use according to the present invention include oral or parenteral administration. Specific examples of the administration methods include administration by injection, transnasal administration, transpulmonary administration, and percutaneous administration. Examples of administration by injection include intravenous injection, intramuscular injection, intraperitoneal injection, and hypodermic injection, by which inducers or tissue regeneration promoters of the present invention can be administered systemically or locally (such as subcutaneously or intracutaneously, or to the skin surface, eyeball, palpebral conjunctiva, rhinal mucosa, intraoral mucosa, gastrointestinal mucosa, vaginal/intrauterine mucosa, or lesion site).

The method of administration may be appropriately selected according to the age and the symptoms of the patient. When an HMGB1, HMGB2, or HMGB3 protein is administered, the dose per time of the protein can be selected within a range of 0.0000001 mg to 1000 mg per kg body weight of a patient. Alternatively, the dose can be selected within a range of 0.00001 mg to 100000mg per body of patient, for example. When administering cells secreting HMGB1, HMGB2, or HMGB3 proteins or gene therapy vectors inserted with DNAs encoding HMGB1, HMGB2, or HMGB3 proteins they may be administered such that the amounts of HMGB1, HMGB2, or HMGB3 protein in the damaged tissues are within the above range. However, the dosage of the tissue regeneration promoters for use according to the present invention are not limited thereto.

Tissue regeneration promoters for use according to the present invention can be formulated according to the usual methods (for example, Remington's Pharmaceutical Science, latest edition, Mark Publishing Company, Easton, U.S.A), and may contain pharmaceutically acceptable carriers and additives together. Examples include surfactants, excipients, colorants, perfumes, preservatives, stabilizers, buffers, suspending agents, isotonizing agents, binders, disintegrants, lubricants, flow promoters, and flavoring agents, although they are not limited thereto and other common carriers may be appropriately used. Specific examples include light anhydrous silicic acid, lactose, crystalline cellulose, mannitol, starch, carmellose calcium, carmellose sodium, hydroxypropylcellulose, hydroxypropylmethylcellulose, polyvinylacetaldiethylamino acetate, polyvinylpyrrolidone, gelatin, medium-chain fatty acid triglyceride, polyoxyethylene hydrogenated castor oil 60, white sugar, carboxymethyl cellulose, corn starch, and inorganic salts.

### Examples

Herein below, the present invention will be described with reference to the Examples, but it is not to be construed as being limited to these Examples.

### [Example 1]

Objective: Assessment of the contribution of bone marrow-derived cells towards functional regeneration of skin tissue transplanted to a living body.

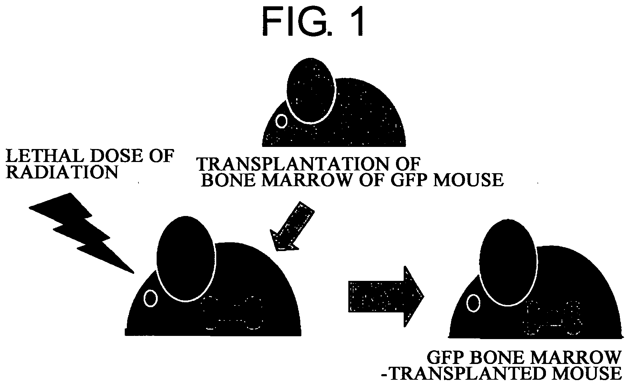

Method: In view of the above objective, studies were carried out by the following methods.
1) Utilizing the live skin transplant system of GFP bone marrow-transplanted mice, the degree of contribution of bone marrow-derived cells towards functional regeneration of grafted skin was examined. Specifically, 6 to 8-week-old male C57BL/6 mice were irradiated with a lethal dose of radiation (10 Gy), and immediately after that, GFP (green fluorescent protein) transgenic mouse-derived bone marrow cells (5x10⁶ cells/0.1ml of physiological phosphate buffer solution at pH 7.4) were transplanted through the caudal vein (Fig. 1)
2) The transplanted bone marrow cells were allowed to engraft (for 6 weeks), and as a result, a GFP bone marrow-transplanted mice was obtained. Then, skin of a neonatal mouse (female) was transplanted to the dorsal skin of the GFP bone marrow-transplanted mice.
3) The skin graft was allowed to engraft and having had satisfactory skin tissue regeneration (4 weeks), the degree of GFP fluorescence accumulation in the grafted skin area was observed using a fluorescence stereoscopic microscope.
4) Under inhalational anesthesia, the skin graft was collected by biopsy. Then, frozen skin sections (6 µm) were prepared using a microtome with a cooling device, and then were fixed with 4% paraformaldehyde (for 30 minutes). Cell nuclei in the tissue were stained with DAPI. Immunostaining was performed using an antibody against epidermal cell-specific keratin 5. The tissue was sealed to examine the presence of GFP-positive bone marrow-derived cells with a confocal laser microscope. A part of the specimen was stained with HE to examine its tissue construction.

Result: In the live skin transplant system of GFP bone marrow-transplanted mice, a strong GFP fluorescence accumulation corresponding to the regenerated skin region was observed (Fig. 2). Moreover, with the histological observation using the HE specimen of the skin graft, functional regeneration of skin tissue containing a large number of hair follicles was observed (Fig. 2). With the observation using a confocal laser microscope, GFP fluorescence was seen in many keratin 5-expressing epidermal keratinocytes, dermal fibroblasts, and further smooth muscle cells and adipocytes, showing that these cells derive from the bone marrow (Fig. 3). That is to say, it was revealed for the first time that many of the epithelial and mesenchymal cells required for functional regeneration of the transplanted skin were supplied from bone marrow-derived stem cells.

Discussion: For the first time, these study results clearly showed a breakthrough discovery that bone marrow-derived cells greatly contribute towards skin regeneration following skin transplantation, which is routine clinical procedure.

It is reported that the bone marrow has two stem cell systems: hematopoietic stem cells and mesenchymal stem cells. It is difficult to imagine that the large number of bone marrow-derived epithelial cells and mesenchymal cells that were mobilized into the transplanted skin (as shown by the present study) were supplied only from bone marrow-derived hematopoietic stem cells. This strongly suggests the possible contribution of bone marrow-derived mesenchymal stem cells towards the functional regeneration of transplanted tissues. That is to say, it was predicted that immediately after skin grafting, a factor for mobilizing bone marrow-derived mesenchymal stem cells is released from the recipient skin tissue heading towards hypoperfusion/necrosis , in which the mesenchymal stem cells are mobilized from the bone marrow through peripheral blood circulation to the transplanted skin piece, and thus inducing functional skin tissue regeneration.

### [Example 2]

Objective: Identification of a bone marrow-derived mesenchymal stem cell-inducing factor in a skin tissue extract

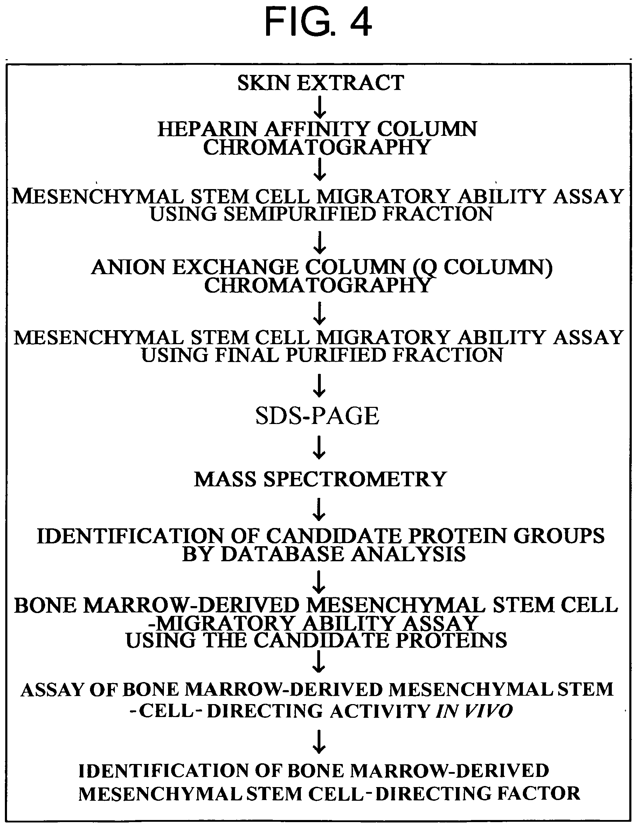

Method: With the objective of identifying a bone marrow-derived mesenchymal stem cell-mobilizing factor which is expected to be released from excised skin under hypoperfusive conditions, studies were carried out by the following methods.
1) To obtain mouse bone marrow-derived mesenchymal stem cells, bone marrow cells were collected from the femur or crus bone of C57BL/6 mice, and then were spread on a cell culture plate having a 10% fetal bovine serum-containing D-MEM (Nacalai) as a cell culture medium, and then were cultured under the condition of 5% CO₂ at 37°C. When the cells proliferated to the point of occupying 70 to 100% of the bottom area of the culture plate, the cells were peeled off from the culture plate using 0.25% trypsin 1m MEDTA (Nacalai), and were then cultured under the above conditions. This passing and culturing procedure was repeated at least five times. Further, these adherent cells were isolated and cultured, followed by an analysis of cell surface antigens using flow cytometry, to confirm that these cells were Lin-negative, CD45-negative, CD44-positive, Sca-1-positive, and c-kit-negative. These cells were confirmed to be able to differentiate into bone cells and adipocytes and have properties of bone marrow mesenchymal stem cells.
2) Free skin pieces obtained from 400 neonatal mice were immersed in 400 ml of physiological phosphate buffer solution at pH 7.4 (PBS). The solution was incubated at 4°C for 24 hours, and then was centrifuged at 440 G at 4°C for 10 minutes to remove the tissue. The supernatant was collected to prepare a skin extract.
3) In order to confirm that the thus obtained skin tissue extract has an activity of inducing bone marrow-derived mesenchymal stem cells, the migration activity of C57BL6 mouse bone marrow-derived mesenchymal stem cells, of which the cell line has been already established by the present inventors, was examined using a Boyden chamber. Specifically, a skin extract (25 µl) was inserted into the lower chamber (volume: 25 µl) of the Boyden chamber, and a polycarbonate membrane having fine pores of 8 µm was placed on it. The upper chamber (volume: 50 µl) of the Boyden chamber was further placed on this in contact, and was filled with a bone marrow-derived mesenchymal stem cell suspension (5 x 10⁴ cells/50 ml of culture solution: DMEM/10% fetal bovine serum). The chamber was incubated in a CO₂ incubator at 37°C for 4 to 24 hours. After culturing, the upper chamber was removed and the silicon membrane was taken out. The number of bone marrow-derived mesenchymal stem cells which had migrated to the lower chamber through the fine pores was quantitatively examined by staining.
4) To purify factors having a bone marrow-derived mesenchymal stem cell-mobilizing activity in the skin extract, heparin affinity column chromatography and anion exchange column (Q column) chromatography were carried out. The skin extract was diluted 10-fold with 9 volumes of 20 mM phosphate buffer at pH 7.5 at 4°C (diluted solution A). 20 mM phosphate buffer at pH 7.5 (30 ml) was poured into HiTrap Heparin HP column (column volume: 5 ml, GE Healthcare) in advance to equilibrate the column. Further, the diluted solution A was allowed to bind to the column. Then, the column was washed with 20 mM phosphate buffer at pH 7.5 with 100 mM NaCl (30 ml). To elute the absorbed proteins, 20 mM phosphate buffer at pH 7.5 with 1000 mM NaCl were poured into the column, to elute the fractions into the tubes. The fractions having the migratory ability according to the migration activity assessment method using a Boyden chamber as described in 2) were collected from each absorbed fraction. This was diluted with 9 volumes of 50 mM Tris HCl pH 8.0 (diluted solution B). 50 mM Tris HCl pH 8.0 (30 ml) was poured into HiTrap mono Q column (column volume: 1 ml, GE Healthcare) in advance to equilibrate the column. Further, the diluted solution B was allowed to bind to the column. In order to elute the absorbed proteins, Tris HCl pH 8.0 and 1000 mM NaCl were poured into the column, to eluate the fractions into tubes. The above purification process can all be performed at 4 to 16°C, but it is preferably 4 to 8°C, and more preferably 4°C. The eluates were assessed by the migration activity assessment method using Boyden chamber as described in 2).
5) The skin extract-derived purified preparations having the bone marrow-derived mesenchymal stem cell-mobilizing activity, which was obtained by combining the migration activity assessment using a Boyden chamber and column chromatography, were subjected to SDS-PAGE electrophoresis to separate within the gel based on the molecular weight, and the bands of migratory proteins were detected by silver staining.
6) Among the skin extract-derived protein groups that had been subjected to SDS-PAGE electrophoresis and that were separated within the gel as single bands by the silver staining of 5), all protein bands obtained from chromatography-purified preparations having the strongest bone marrow-derived mesenchymal stem cell-mobilizing activity were excised, and then the identification of these proteins by mass spectrometry and database analysis was carried out.
7) Among the identified protein groups, candidate proteins having the bone marrow-derived mesenchymal stem cell-mobilizing activity were selected. Purified preparations including such candidate proteins were treated with neutralizing antibodies (100 µl of purified preparation solution was incubated on ice for 30 minutes with 100-fold diluted polyclonal antibody of the candidate protein. Then, the degree of inhibition on the bone marrow-derived mesenchymal stem cell-mobilizing activity was examined by migratory ability assessment using a Boyden chamber.
8) The obtained purified bone marrow-derived mesenchymal stem cell preparations were mixed in Matrigel at about 10% volume. A silicon tube having a diameter of about 1 mm and a length of 5 mm was filled with the Matrigel, which was then subcutaneously transplanted to the back of GFP bone marrow-transplanted mouse. Two weeks after, the inserted tube was taken out, and GFP fluorescence emitting from bone marrow-derived cells which had migrated into the tube was quantitatively analyzed by a fluorimeter. Further, the migratory cells were taken out from the tube, and were inoculated into a DMEM/10% fetal bovine serum medium, followed by culturing in a CO₂ incubator, to examine the *in vivo* bone marrow-derived mesenchymal stem cell-mobilizing activity. These cells that were continuously cultured for 2 weeks were fixed with 2% paraformaldehyde at 25°C for 10 minutes, and rinsed with PBS four times, 5 minutes each, to wash out the paraformaldehyde. Then this was treated with a 2% skim milk solution, and was allowed to react with 1000-fold dilution of anti-mouse keratin 5 antibody (diluted with 2% skim milk containing 0.5% tween 20) at 4°C for 16 hours. The antibody was washed out with PBS four times for 5 minutes each. This was then allowed to react with 1000-fold diluted Alexa546-labelled anti-rabbit IgG antibody (diluted with 2% skim milk) at 25°C for 1 hour. The above experimental protocol of 1) to 8) is summarized in Fig. 4.

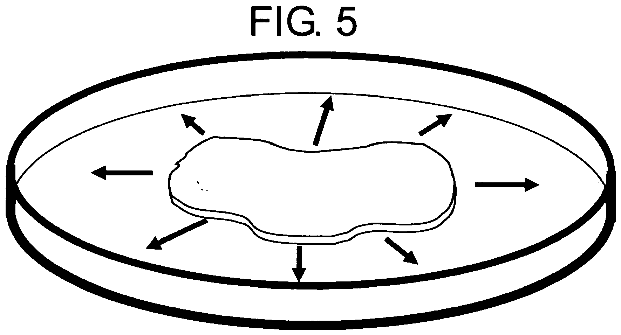

Result: Starting from the extract solution of excised skin of neonatal mouse in PBS (Fig. 5), proteins having the bone marrow-derived mesenchymal stem cell-mobilizing activity was subjected to identification and functional analysis by the above-mentioned methods. The migration activity assessment using a Boyden chamber showed that the skin extract has an extremely strong bone marrow-derived mesenchymal stem cell-inducing activity (Fig. 6). Using this activity as an index, a heparin affinity column and an anion exchange column (Q column) were used to proceed with the purification of the target factor. The obtained fractions were each analyzed by SDS-PAGE electrophoresis. As a result, a strong bone marrow-derived mesenchymal stem cell-mobilizing activity was shown by silver staining in the purified preparation containing several proteins that were separated within the gel in the form of single bands (Lane 7 in Fig. 7). The obtained silver-stained bands were excised, and were then subjected to mass spectrometry and database analysis. As a result, it was revealed that the protein having a molecular weight of about 25,000 indicated by the arrow was HMGB1 (Fig. 7). To clarify that HMGB1 contained in this purified fraction (Lane 7) has the intended bone marrow-derived mesenchymal stem cell-mobilizing activity, a migration inhibition experiment was carried out using an anti-MGB1 polyclonal antibody. As a result, it was revealed that the anti-HMGB1 polyclonal antibody strongly inhibits the bone marrow-derived mesenchymal stem cell migration activity in the purified preparation (Fig. 8) and that the bone marrow-derived stem cell-mobilizing factor present in the skin extract is HMGB1.

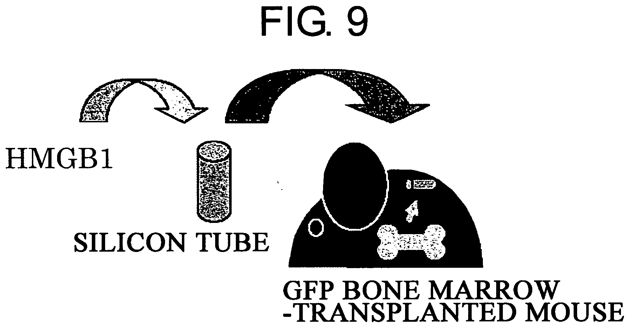

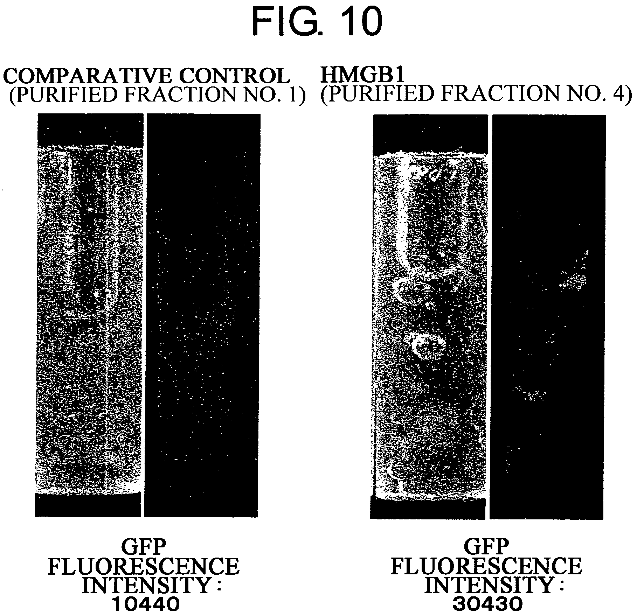

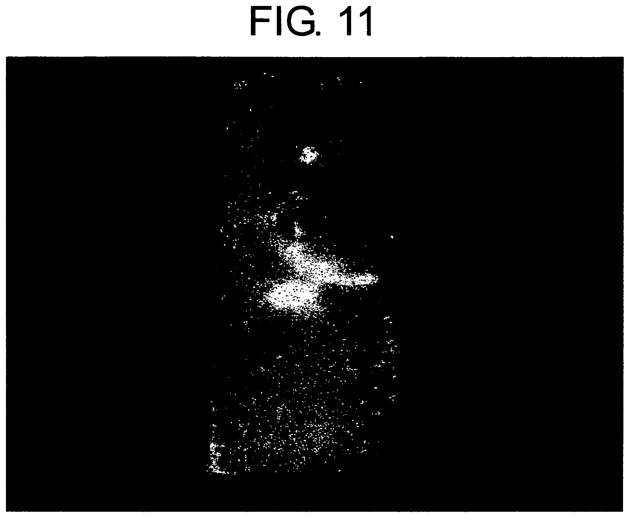

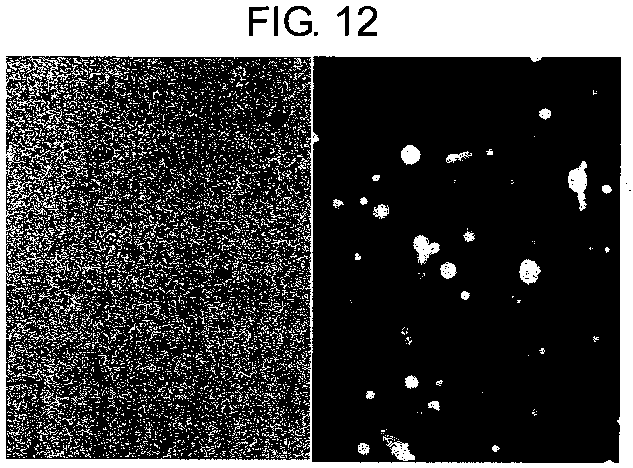

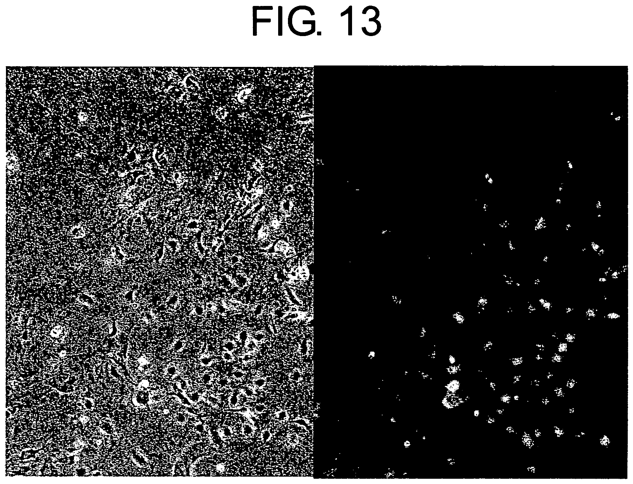

Further, to confirm that HMGB1 has a bone marrow-derived mesenchymal stem cell-mobilizing activity *in vivo*, a silicon tube containing this purified preparation was subcutaneously inserted into the back of GFP bone marrow-transplanted mouse. Two weeks after, the properties of cells mobilized into the tube were examined (Fig. 9). As a result, the HMGB1 purified preparation mobilized a greater number of GFP-positive bone marrow-derived cells into the tube (about three times) as compared to the comparative control (purified preparation used for Lane 4 in SDS-PAGE of Fig. 7) (Fig. 10). Fig. 11 shows a high magnification image by a fluorescence stereoscopic microscope. Further, GFP-positive cells mobilized into the tube were taken out, and were cultured in a DMEM/10% fetal bovine serum medium. As a result, round-shaped floating cells were observed immediately after culturing (Fig. 12), however 24 hours after the GFP-positive bone marrow-derived cells were confirmed to adhere onto the culture dish and proliferated in the form of spindle-shaped fibroblast-like cells and further in the form of cylindroid-shaped epithelial-like cells (Fig. 13). When these cells were continuously cultured for another 2 weeks, hair follicle-forming cells were observed among the GFP-positive bone marrow-derived cells (Fig. 14A; light field, low magnification, Fig. 14B; GFP fluorescence, low magnification, Fig. 14C; light field, high magnification, Fig. 14D; GFP fluorescence, high magnification). Moreover, when immunohistochemical techniques were used for keratin 5, a marker for epithelial keratinocytes, keratin 5-positive cells were observed among the GFP-positive bone marrow-derived cells (Fig. 14E; light field, Fig. 14F; fluorescence of keratin 5-positive cells).

Discussion: This time, the present inventors have discovered for the first time in the world that: free skin pieces produce HMGB1; the produced HMGB1 has an activity of mobilizing a large amount of bone marrow-derived mesenchymal stem cells into the skin pieces; bone marrow-derived mesenchymal stem cells mobilized into the skin pieces are differentiated into mesenchymal cells such as fibroblasts, adipocytes, smooth muscle cells in the skin tissue, and further are differentiated into cells that form hair follicles of epidermal cells, to induce functional regeneration of transplanted skin tissues. It can be readily predicted that this mobilization of bone marrow-derived mesenchymal stem cells by HMGB1 and the resulting functional tissue regeneration functions, not only for transplanted skin regeneration, but also as a mechanism for inducing functional tissue regeneration in various damaged organs/tissues accompanying hypoperfusion/necrosis. The present inventors firmly believe that, if drug development using an HMGB1 formulation enables the mobilization of bone marrow-derived mesenchymal stem cells to the local area during regeneration of the damaged tissues, it would enable functional tissue regeneration-inducing therapy for vital functional organs, without the organs becoming dysfunctional due to fibrous scar healing.

### [Example 3]

Objective: Identification of the HMGB1 family in the skin extract and examination of bone marrow mesenchymal stem cell-inducing activity

Method: Whether or not the neonatal mouse skin extract contained the HMGB protein family was confirmed using the Western blot method. Ten µl of the skin extract obtained in [Example 2] was used as a sample and subjected to SDS-PAGE electrophoresis. The proteins separated within the gel were transferred onto a PVDF membrane using a blotting device (ATTO). The membrane was incubated with PBS containing 3% skim milk and 0.1% Tween 20 (S-T-PBS) at room temperature for 1 hour, and then was allowed to react with each of rabbit anti-mouse HMGB1 antibody, rabbit anti-mouse HMGB2 antibody, or rabbit anti-mouse HMGB3 antibody which were diluted 1000-fold with S-T-PBS, at 4°C for 16 hours. After the reaction, the PVDF membrane was washed with S-T-PBS five times for 5 minutes. Then, the PVDF membrane was incubated with 2000-fold diluted (diluted with S-T-PBS) peroxidase labeled goat anti-rabbit IgG antibody (GE Healthcare)at 25°C for 1 hour. Further, after washing with S-T-PBS five times for 5 minute, the PVDF membrane was allowed to react with ECL Western Blotting Detection System (GE Healthcare). The ECL film was exposed and developed to detect the presence of HMGB 1, HMGB2, and HMGB3 proteins.

RNA was extracted from the skin of neonatal mouse using Trizol (invitrogen), and further cDNA was synthesized using SuperScript III cDNA synthesis kit (Invitrogen). Using this cDNA as a template, cDNAs of HMGB 1, HMGB2, and HMGB3 were amplified using the PCR (polymerase chain reaction) method. The cDNAs were inserted into the plasmid vector pCAGGS for expressing proteins in mammalian cells, such that proteins with an additional Flag tag sequence (Asp-Tyr-Lys-Asp-Asp-Asp-Lys; SEQ ID: 18) at the N terminus of the amino acid sequence could be expressed. These plasmid vectors were introduced into HEK293 (Human embryonic kidney derived culture cell line) and cultured for 48 hours to express the proteins. Cells expressing each of the HMGB1, HMGB2, and HMGB3 proteins and the culture supernatant were incubated at 4°C for 16 hours, which was then centrifuged at 4400 g for 5 minutes to collect the supernatant. 100 µL of the anti-Flag antibody gel (Sigma) was mixed into 50 mL of this supernatant, and was then incubated at 4°C for 16 hours. Centrifugation was then performed to collect the gel, and washed with PBS five times. Further, the protein was eluted using 3X Flag peptide (final 100 µg/ml). Expressions of recombinant proteins were observed by the Western blot method using 1000-fold diluted (diluted with S-T-PBS) mouse anti-Flag antibody and 2000-fold diluted (diluted with S-T-PBS) peroxidase-labeled anti-mouse IgG antibody (GE Healthcare). The mouse bone marrow mesenchymal stem cell migration activity in these purified recombinant proteins was assessed in the same manner as in [Example 2] using a Boyden chamber. Moreover, in order to observe the *in vivo* drug efficacy of the HMGB family, the dorsal skin of 8-week-old C57BL/6 mice was cut out in a circle having a diameter of 8 µm to prepare cutaneous ulcer models. Purified HMGB1, HMGB2, and HMGB3 (100 ng) were each mixed with the same amount of hyaluronic acid solution having a concentration of 1 g/100 mL of PBS, and 100 µL of it was administered to the ulcer surface. The ulcer surface was covered with a transparent adhesive wound dressing/protective material Tegaderm (3M Healthcare) to avoid drying, and the wound area was measured over time to determine the therapeutic effect.

Further, to examine whether or not the human skin extract and the purified human HMGB1 has an activity to allow migration of human bone marrow mesenchymal stem cells, a Boyden chamber was used in the same manner as in [Example 2] for assessment. A human skin having an area of 1cm² was immersed in 1 ml PBS, and then was incubated at 4°C for 16 hours and subsequently centrifuged at 440 G at 4°C for 10 minutes. The supernatant alone was collected to be used as a human skin extract. Moreover, human bone marrow mesenchymal stem cells (Cambrex) were used as the cells to be placed in the upper chamber of the Boyden chamber (as a result of surface antigen analysis by flow cytometry, these cells have been confirmed to be CD105-positive, CD166-positive, CD29-positive, CD44-positive, CD34-negative, and CD45-negative. They have also been found to differentiate into adipocytes, chondrocytes, and bone cells by differentiation induction tests). Moreover, 100 ng/well of human HMGB1 (R&D) and human skin extract diluted 10-fold with PBS and were placed in the lower chamber. PBS was used as a control.

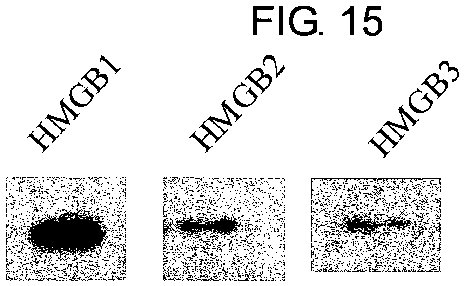

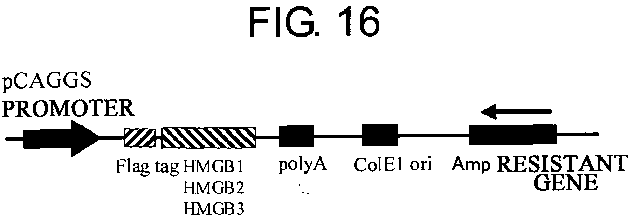

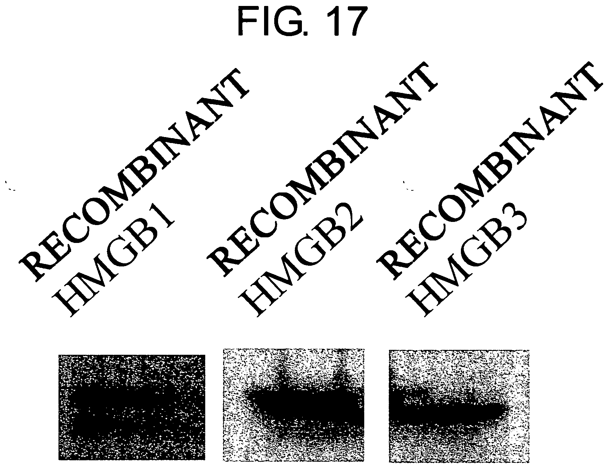

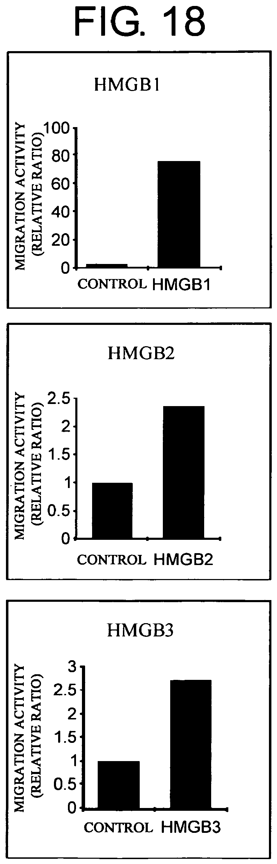

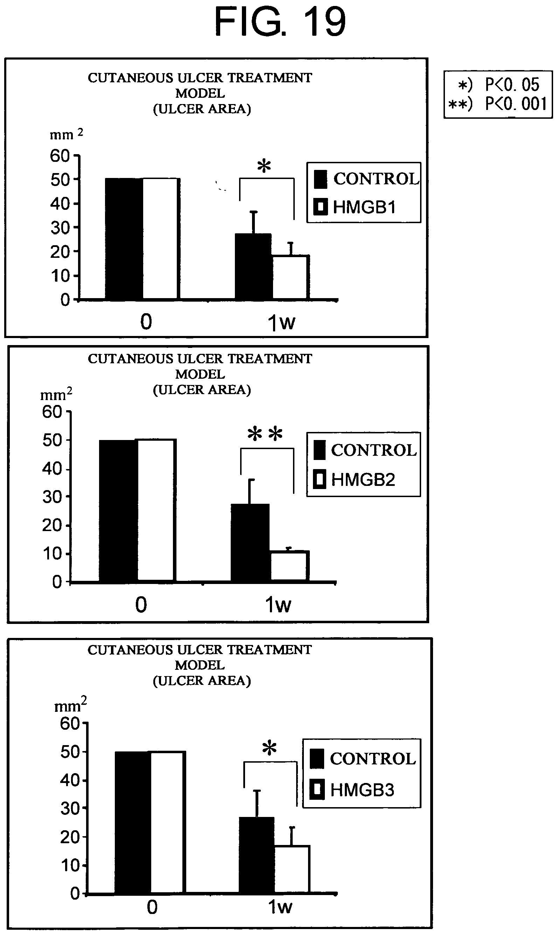

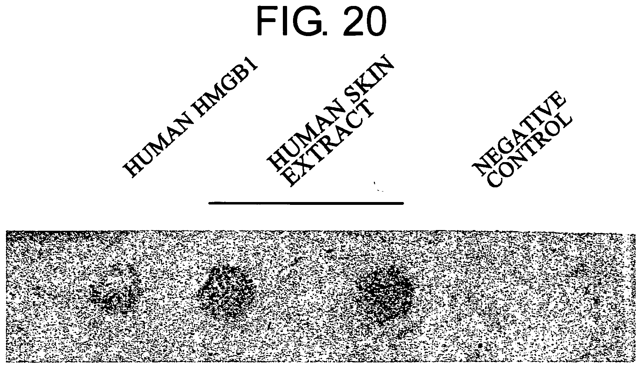

Result: As a result of Western blotting, bands of HMGB2 and HMGB3 were detected as well as the HMGB1 band. Therefore, the neonatal mouse skin extract was confirmed to contain the family proteins, HMGB2 and HMGB3, besides HMGB1 (Fig. 15). Expression vectors of HMGB1/HMGB2/HMGB3 having a Flag tag added at the N-terminus of each protein, were prepared (Fig. 16). These expression vectors were introduced into HEK293 cells, and the expressed proteins were purified using the Flag tag, and Western blotting was carried out to observe these proteins (Fig. 17). The mouse bone marrow mesenchymal stem cell migration activity was measured using these purified proteins, and the activity was confirmed in all of the proteins (Fig. 18). The ulcer area produced in the back of the mouse was measured every 7 days, and a significant effect on reducing ulcer area was confirmed in the HMGB1, 2, and 3 treatment group, as compared to the non-treatment group (Fig. 19). Similar to the mouse case, human HMGB1 and the human skin extract were revealed to have human bone marrow mesenchymal stem cell migration activity (Fig. 20).

Discussion: HMGB2 and HMGB3 are known as proteins having high homologies to HMGB1. These proteins are also expected to have properties similar to HMGB1. It was confirmed that HMGB2 and HMGB3 of the HMGB1 family are also produced from the extract of the free skin section. Further, HMGB1/HMGB2/HMGB3 recombinant proteins were produced, and their *in vitro* bone marrow mesenchymal stem cell migration activity and the *in vivo* therapeutic effect on a cutaneous ulcer were also confirmed. It was revealed that the HMGB family (HMGB1/HMGB2/HMGB3) and the recombinant HMGB family in the neonatal mouse free skin section have a bone marrow mesenchymal stem cell-inducing activity and an activity of locally inducing bone marrow-derived stem cells which are differentiatable into epithelium, and that the thus induced bone marrow-derived cell group differentiates into various cells such as epidermal keratinocytes, hair follicles, and fibroblasts in the damaged tissue to promote the recovery of the damaged tissue. Moreover, since bone marrow mesenchymal stem cells are multipotent stem cells, the present inventors believe that therapeutic effects can also be expected in the same manner by systematic administration or local administration of the HMGB family to treat damaged states in other tissues, for example, tissue damages such as brain injury, myocardial infarction, and bone fracture.

Moreover, it is known that, between human and mouse, amino acid sequence homology for HMGB1 is 98% (213/215), 96% (202/210) for HMGB2, and 97% (195/200) for HMGB3. Therefore, human HMGB and mouse HMGB are considered to have similar activities, and the results of the present Examples revealed that human skin extract and human HMGB1 have bone marrow mesenchymal stem cell-inducing activities in a manner same as those of mouse skin extract and mouse HMGB 1.

### [Example 4]

Objective: Establishment of a method of producing a tissue extract containing bone marrow mesenchymal stem cell-inducing factors.

Method: Brain, heart, intestine, kidney, and liver of a 6-week-old C57BL6 mouse and skin of a neonatal mouse were immersed in 1 ml of physiological phosphate buffer solution (PBS) at pH 7.4. The solutions were incubated at 4°C for 24 hours, and then centrifuged at 440 G at 4°C for 10 minutes to remove the tissues. The supernatants were collected to prepare tissue extracts. To confirm whether the thus obtained extract has a bone marrow-derived mesenchymal stem cell-inducing activity, the migration activity of bone marrow-derived mesenchymal stem cells was examined in the same manner as in [Example 2] using a Boyden chamber. Moreover, the HMGB1 concentration contained in these samples was measured using an HMGB1 ELISA kit (Shino-Test). Further, tissue extracts of the brain, heart, and skin were allowed to bind to a heparin affinity column in the same manner as in [Example 2], and the bone marrow-derived mesenchymal stem cell-inducing activity in the protein-bound fraction was confirmed using Boyden chamber.

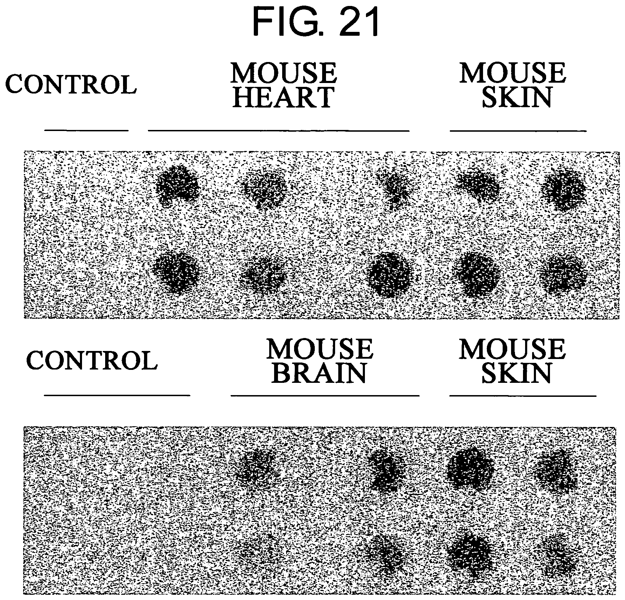

Result: The mouse brain extract contained an amount of HMGB1 equivalent to the neonatal mouse skin extract. Further, bone marrow mesenchymal stem cell-inducing activity was also observed in the mouse brain as well as in the skin. Although the mouse intestine extract and the mouse heart extract contained little HMGB1, bone marrow mesenchymal stem cell-inducing activities were observed. Moreover, the heparin column-bound fractions of mouse brain and mouse heart, as well as the heparin column-bound fraction of mouse skin, showed bone marrow mesenchymal stem cell-inducing activities (Fig. 21). Table 1 shows the measurement results of the HMGB1 concentration and the bone marrow mesenchymal stem cell-inducing activity in each of the mouse tissue extracts.

**[Table 1]**

| | **HMGB1** concentration **(ng/mL)** | Bone marrow |
|---|---|---|
| | | -mesenchymal stem cell |
| | | -inducing activity |
| Skin | 110 | Present |
| Brain | 140 | Present |
| Heart | 4 | Present |
| Intestine | 0 | Present |
| Kidney | 115 | ND |
| Liver | 61 | ND |

| | | |
|---|---|---|
| ND: No Data | | |

Discussion: A method in which HMGB1 can be conveniently extracted not only from the skin but also from the brain was developed by simply immersing these organs in a physiological buffer. This method is also applicable to other organs such as liver and kidney. Moreover, although the extracts from intestine and heart contain little HMGB1, a bone marrow mesenchymal stem cell-inducing activity was observed. This suggests these extracts contain other bone marrow mesenchymal stem cell-inducing substance(s) apart from HMGB1. Such substances contained in these extracts are originally present in each tissue, and are considered to physiologically induce bone marrow mesenchymal stem cells to the damaged tissue when the tissue is damaged. The present invention developed a novel method for conveniently and functionally extracting multiple bone marrow mesenchymal stem cell-inducing substances including HMGB1, from various organs. Further, a method for purifying bone marrow mesenchymal stem cell-inducing substances from a tissue extract using the binding to the heparin column was also developed. These substances having bone marrow mesenchymal stem cell-inducing activities can be purified from the brain and heart in the same manner as in the skin using a heparin column.

### [Example 5]

Objective: Establishment of a method for extracting mesenchymal stem cell migration activators from cultured cells.

Method: Human embryonic kidney derived cultured cell line HEK293 and human cervix carcinoma cell line HeLa were each cultured in 10% fetal bovine serum-containing D-MEM (Nacalai). These cells were each washed with PBS, and then 10⁷ cells were immersed in 5 ml of PBS (Nacalai) at 4°C for 16 hours. The solution was centrifuged at 440 G (acceleration of gravity) at 4°C for 5 minutes, and then the supernatant was collected. Human bone marrow mesenchymal stem cells were placed in the upper chamber of a Boyden chamber, and a 5-fold diluted (with DMEM) cell extract was placed in the lower chamber, to confirm the migration activity of human bone marrow mesenchymal stem cells.

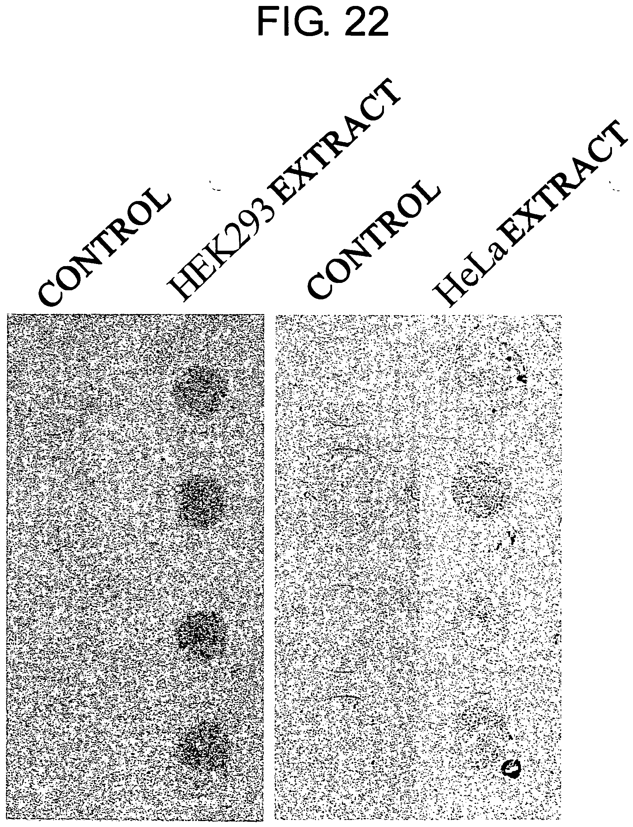

Result: HEK293 extract and HeLa extract both showed similar bone marrow mesenchymal stem cell migration activities(Fig. 22).

Discussion: Bone marrow mesenchymal stem cell migration activators were successfully extracted by the convenient method of immersing cultured cells in PBS.

### [Example 6]

Objective: Whether or not regeneration of neural cells can be induced is examined by producing mouse brain-defective models, to which a heparin-column purified fraction of skin extract is administered in a sustained-release manner at the local lesion site, by which stem cells contained in a mouse myeloid system is allowed to migrate into the local lesion site.

### Method:

### (1) Preparation of heparin-column purified fraction of skin extract

An excised skin section of a neonatal mouse was incubated in PBS (mouse/ml) at 4°C for 16 hours, and a skin extract was obtained. The skin extract was diluted 10-fold with 9 volumes of 20 mM phosphate buffer at pH 7.5 at 4°C. 20 mM phosphate buffer at pH 7.5 (30 ml) was poured into HiTrap Hepalin HP column (column volume: 5 ml, GE Healthcare) in advance to equilibrate the column. The diluted solution was then allowed to bind to the column. Thereafter, the column was washed with 20 mM phosphate buffer at pH 7.5 and 100 mM NaCl (30 ml). To elute the absorbed proteins, 20 mM phosphate buffer at pH 7.5 and 1000 mM NaCl were poured into the column, and the factions were eluted into the tubes. Each of the absorbed factions were evaluated according to the mouse bone marrow-derived cell migration activity assessment using the Boyden chamber method shown in Example 2, and fraction(s) having migratory activity was collected. Solution(s) having the activity was used as a heparin purified fraction(s) of the skin extract.

### (2) Production of myelosuppressive mice

Mice were irradiated with single-dose of X ray at 10 Gy to produce myelosuppressive mice.

### (3) Transplant of GFP mouse bone marrow to myelosuppressive mice

Bone marrow cells were collected from both femurs and crus bones of GFP mice. These cells were administered to the myelosuppressive mice through the caudal vein 24 hours after the irradiation. The administration was carried out under inhalational anesthesia using isoflurane.

### (4) Production of a brain-defective (brain tissue-defective) mouse model

The myelosuppressive mice transplanted with GFP mouse bone marrow cells were subjected to inhalational anesthesia using isoflurane, and pentobarbital (45 mg/kg) was intraperitoneally injected to the mice. The mice were fixed onto a brain stereotaxis apparatus and subjected to a midline incision in the head with a scalpel. Trepanation was carried out at 2.5 mm right-lateral and 12.5 mm anterior to the bregma using a drill (Fig. 23A). At a 3 mm depth from this site, a 20G Surflow needle was inserted and fixed. Then, a negative pressure was applied using a syringe to suck a part of the brain tissue (Fig. 23B).

### (5) Administration of a heparin-column purified fraction of skin extract to the brain tissue-defective site

Five µl of a heparin-column purified fraction of skin extract dissolved in fibrinogen of the fibrin tissue adhesive Bolheal (Kaketsuken) was injected to the above site, and subsequently, 5 µl of thrombin of Bolheal was injected using a Hamilton syringe and a 26G syringe (Fig. 23C). The aim of this operation was to exert the sustained-release agent effect of a heparin-column-purified fraction of the skin extract.

### (6) Assessment of the effects of neural cell regeneration in brain tissue-defective sites

Mice of the control group and the treatment group were used for the assessment. An appropriate elapsed time setting (over time) was determined, the mice were perfused with 4% paraformaldehyde and fixed and then the brain was cut out. Further, external fixation was performed with 4% paraformaldehyde. These were then dehydrated in a 15% and 30% sucrose gradient to produce frozen sections.

The nucleus were stained with a DAPI (4',6-Diamidino-2-phenylindole, dihydrochloride) solution and the section was sealed using a photobleaching inhibitor. The accumulation of GFP-positive cells in the lesion site (brain tissue-defective site) was assessed using a confocal laser microscope.

Result: The accumulation of GFP-positive cells is qualitatively shown for 2 weeks, and 6 weeks after the administration. The accumulation of GFP-positive cells tend to be higher in the lesion sites of the treatment group rather than the control group, for both 2 weeks (control; Fig. 23D, skin extract heparin-column-purified fraction; Fig. 23E) and 6 weeks (control; Fig. 23F, skin extract heparin-column-purified fraction; Fig. 23G) after the administration.

Discussion: The administration of the heparin-column-purified fraction of the skin extract resulted in the accumulation of bone marrow-derived cells in the brain tissue-defective site, which showed a nerve cell form. Bone marrow-derived mesenchymal stem cells are also known to differentiate into nerve cells and the result revealed that the heparin-column purified fraction of the skin extract is capable of inducing neural cell regeneration of the injured site in the brain. Moreover, this is also applicable to neuronal regeneration of damaged sites in brain tissues in cerebral ischemic diseases and cerebral contusions.

### Industrial Applicability

Tissue regeneration promoters comprising HMGB1, HMGB2, or HMGB3 are provided. The development of HMGB1, HMGB2, or HMGB3 in the form of bone marrow-derived cell-mobilizing drugs enable novel regenerative medicines which promote functional regeneration of intractable damaged tissues. HMGB1, HMGB2, and HMGB3 are released from cells/tissues heading towards necrosis due to a low-oxygen state caused by a decreased blood circulation, and have an effect of locally mobilizing bone marrow-derived cells or other various cells derived therefrom to such cells/tissues. The mobilized bone marrow-derived cells are differentiated into cell lineages required in each tissue, and promote the recovery of functions lost due to hypoperfusion/necrosis, namely promote the so-called functional tissue regeneration. For example, the administration of HMGB1, HMGB2, or HMGB to an intractable cutaneous ulcer caused by blood circulation disorders of the skin leads to mobilization of bone marrow-derived cells to the ulcer surface. Once the mobilized cells are differentiated into vascular endothelial cells, the local blood circulation would be improved. At the same time, differentiation into fibroblasts, nerve cells, hair follicle cells, and further epidermal cells would lead to the induction and promotion of functional regeneration of skin with necessary dermal appendages, rather than just healing with fibrous scar tissues. HMGB1, HMGB2, and HMGB3 are expected to have a similar effect as functional tissue regeneration inducers for myocardial infarction, cerebral infarction, and hypoperfusive necrosis states of other organs.

## Claims

1. A tissue regeneration promoter comprising any one of the following components (a) to (i) for use in regenerative medicine for epithelial or neurological tissue:
(a) an HMGB1 protein;
(b) a cell secreting an HMGB1 protein;
(c) a vector inserted with a DNA encoding an HMGB1 protein;
(d) an HMGB2 protein;
(e) a cell secreting an HMGB2 protein;
(f) a vector inserted with a DNA encoding an HMGB2 protein;
(g) an HMGB3 protein;
(h) a cell secreting an HMGB3 protein; and
(i) a vector inserted with a DNA encoding an HMGB3 protein.

2. A kit comprising any one of the following components (a) to (i) for use in regenerative medicine for epithelial or neurological tissue:
(a) an HMGB1 protein;
(b) a cell secreting an HMGB1 protein;
(c) a vector inserted with a DNA encoding an HMGB1 protein;
(d) an HMGB2 protein;
(e) a cell secreting an HMGB2 protein;
(f) a vector inserted with a DNA encoding an HMGB2 protein;
(g) an HMGB3 protein;
(h) a cell secreting an HMGB3 protein; and
(i) a vector inserted with a DNA encoding an HMGB3 protein.

## Patentansprüche

1. Geweberegenerationsförderer umfassend eine der folgenden Komponenten (a) bis (i) zur Verwendung in der regenerativen Medizin für epitheliales oder neurologisches Gewebe:
(a) ein HMGB1-Protein;
(b) eine Zelle, die ein HMGB1-Protein sezerniert;
(c) einen Vektor, der mit einer für ein HMGB1-Protein kodierenden DNA insertiert ist;
(d) ein HMGB2-Protein;
(e) eine Zelle, die ein HMGB2-Protein sezerniert;
(f) einen Vektor, der mit einer für ein HMGB2-Protein kodierenden DNA insertiert ist;
(g) ein HMGB3-Protein;
(h) eine Zelle, die ein HMGB3-Protein sezerniert; und
(i) einen Vektor, der mit einer für ein HMGB3-Protein kodierenden DNA insertiert ist.

2. Kit umfassend eine der folgenden Komponenten (a) bis (i) zur Verwendung in der regenerativen Medizin für epitheliales oder neurologisches Gewebe:
(a) ein HMGB1-Protein;
(b) eine Zelle, die ein HMGB1-Protein sezerniert;
(c) einen Vektor, der mit einer für ein HMGB1-Protein kodierenden DNA insertiert ist;
(d) ein HMGB2-Protein;
(e) eine Zelle, die ein HMGB2-Protein sezerniert;
(f) einen Vektor, der mit einer für ein HMGB2-Protein kodierenden DNA insertiert ist;
(g) ein HMGB3-Protein;
(h) eine Zelle, die ein HMGB3-Protein sezerniert; und
(i) einen Vektor, der mit einer für ein HMGB3-Protein kodierenden DNA insertiert ist.

## Revendications

1. Promoteur de régénération de tissu comprenant l'un quelconque des composants (a) à (i) suivants, pour une utilisation en médecine régénérative pour un tissu épithélial ou neurologique :
(a) une protéine HMGB1 ;
(b) une cellule sécrétant une protéine HMGB1 ;
(c) un vecteur inséré avec un ADN codant pour une protéine HMGB1 ;
(d) une protéine HMGB2 ;
(e) une cellule sécrétant une protéine HMGB2 ;
(f) un vecteur inséré avec un ADN codant pour une protéine HMGB2 ;
(g) une protéine HMGB3 ;
(h) une cellule sécrétant une protéine HMGB3 ; et
(i) un vecteur inséré avec un ADN codant pour une protéine HMGB3.

2. Kit comprenant l'un quelconque des composants (a) à (i) suivants, pour une utilisation en médecine régénérative pour un tissu épithélial ou neurologique :
(a) une protéine HMGB1 ;
(b) une cellule sécrétant une protéine HMGB1 ;
(c) un vecteur inséré avec un ADN codant pour une protéine HMGB1 ;
(d) une protéine HMGB2 ;
(e) une cellule sécrétant une protéine HMGB2 ;
(f) un vecteur inséré avec un ADN codant pour une protéine HMGB2 ;
(g) une protéine HMGB3 ;
(h) une cellule sécrétant une protéine HMGB3 ; et
(i) un vecteur inséré avec un ADN codant pour une protéine HMGB3.
